(19) Europäisches Patentamt
European Patent Office
Office européen des brevets

(11) Publication number: 0 085 959
B1

(12) **EUROPEAN PATENT SPECIFICATION**

(45) Date of publication of patent specification: 19.04.89

(21) Application number: 83101036.8

(22) Date of filing: 03.02.83

(60) Divisional application 87108671 filed on 16.06.87.

(51) Int. Cl.$^4$: C 07 D 213/55, C 07 D 213/56, C 07 D 295/14, C 07 D 213/38, C 07 D 213/48, C 07 D 213/79, C 07 C 101/28, A 61 K 31/40, A 61 K 31/44

(54) **Aromatic compounds.**

(30) Priority: 04.02.82 GB 8203261
18.10.82 GB 8229705

(43) Date of publication of application:
17.08.83 Bulletin 83/33

(45) Publication of the grant of the patent:
19.04.89 Bulletin 89/16

(84) Designated Contracting States:
AT BE CH DE FR GB IT LI LU NL SE

(56) References cited:
DE-B-1 227 464
GB-A- 807 757
US-A-2 567 245

Chemical Abstracts vol. 71, no. 3, 21 July 1969, Columbus, OH (US); R.KUNTZMAN et al.: "Methodology for studies of drug metabolism", p. 171, no. 11415f

The file contains technical information submitted after the application was filed and not included in this specification

(73) Proprietor: THE WELLCOME FOUNDATION LIMITED
183-193 Euston Road
London NW1 2BP (GB)

(72) Inventor: Coker, Geoffrey George
80 Pickhurst Park
Bromley Kent (GB)
Inventor: Findlay, John William Addison
Bt.2, Box 514, Cascade Drive
Chapel Hill, NC 27514 (US)

(74) Representative: Sandmair, Kurt, Dr. et al
Patentanwälte Schwabe, Sandmair, Marx
Stuntzstrasse 16
D-8000 München 80 (DE)

(56) References cited:
Medicinal Chemistry, 3rd edition, A. Burger, page 76

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 085 959 B1

**Description**

The present invention relates to new chemical compounds exhibiting anti-histamine activity, to processes for preparing them, to novel intermediates involved in their preparation, to pharmaceutical compositions containing them and to their use in medicine.

US Patent No. 2567245 discloses a group of pyridyl aliphatic amines with antihistamine activity and specially discloses 3-(p-bromophenyl)-3-(2-pyridyl)-N,N-dimthylpropylamine and 3-(p-chlorophenyl)-3-(2-pyridyl)-N,N-dimethylpropylamine which are hereinafter referred to by their generic names brompheniramine and chlorpheniramine respectively.

US Patent 2,712,023 discloses a group of pyridyl propenylamines with antihistamine activity, the most outstanding of which is the compound named (*E*)-1-(4-methylphenyl)-1-(2-pyridyl)-3-pyrrolidinoprop-1-ene and hereinafter referred to by its generic name, triprolidine. Triprolidine has gained widespread clinical acceptance and is one of the most potent antihistamines available.

Triprolidine is known to be metabolized in man to (*E*)-1-(4-carboxyphenyl)-1-(2-pyridyl)-3-pyrrolidinoprop-1-ene which has little or no antihistamine activity.

The antihistamines now in use, including diphenylhydramine, the pheniramines, pyrilamine, promethazine and triprolidine have one potential disadvantage in common; they all cause sedation or drowsiness in some patients.

A novel group of compounds having antihistamine activity has now been discovered.

Accordingly this invention provides a compound of the formula (I).

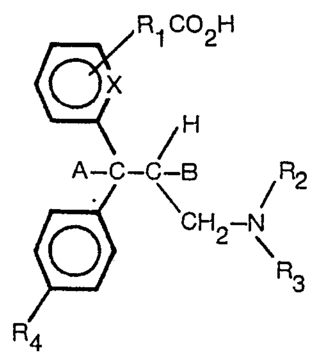

(I)

or a salt, ester or amide thereof; wherein

$R_1$ is a $C_{1-7}$ bivalent aliphatic hydrocarbon group or a single bond;

$R_2$ and $R_3$ are the same or different and are each hydrogen, $C_{1-4}$ alkyl or taken together with the nitrogen comrise a nitrogen-containing heterocyclic ring having four to six ring members;

$R_4$ is hydrogen, halogen, hydroxy, cyano, $C_{1-4}$ acyloxy, $C_{1-4}$ alkoxy or $C_{1-4}$ alkyl optionally substituted by one to three halogen atoms;

X is —N= or —CH=; and

A and B each represent hydrogen atoms or —CA—CB— represents —C=C—.

Of the compounds of formula (I) those of formula (II) are preferred.

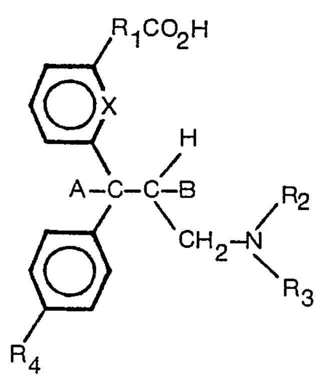

(II)

or a salt, ester or amide thereof; wherein $R_1$ to $R_4$, X, A and B are as defined in relation to formula (I).

$R_1$ may be a straight or branched chain, saturated or unsaturated hydrocarbon group or a single bond. Suitably $R_1$ is a straight chain hydrocarbon group or a single bond. Suitably $R_1$ contains at the most one double or triple bond. Preferably $R_1$ is a group $(CH_2)_n$ wherein n is an integer 0 to 7, or a group $(CH_2)_a$ $CH{=}CH(CH_2)_b$ are independently 0 to 5 and the sum of a and b does not exceed 5.

Further preferred compounds of the formula (I) include those of the formula (IIIa) or (IIIb)

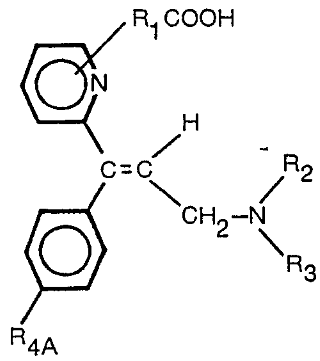

(IIIa)

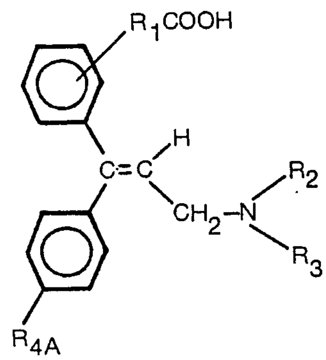

(IIIb)

or a salt, ester or amide thereof;

wherein $R_1$ is $(CH_2)_n$, where n is an integer 1 to 7, or $(CH_2)_aCH:CH(CH_2)_b$, where a and b are independently 0 to 5 and the sum of a and b does not exceed 5; $R_2$ and $R_3$ are the same or different and are hydrogen, lower alkyl (1 to 4 carbons) or taken together with the nitrogen comprise a nitrogen-containing heterocyclic ring (of four to six ring members) such as pyrrolidino, piperidino or morpholino; and $R_{4A}$ is hydrogen, halogen, lower alkyl (1 to 4 carbons) or lower alkoxy (1 to 4 carbons).

Suitably n is 0 to 3 and preferably n is 2. Suitably the sum of a and b does not exceed 2 and preferably a and b are both 0.

Suitably $R_2$ and $R_3$ are the same or different and each is methyl or ethyl or taken together with the nitrogen atom to which they are attached form a four to six membered heterocyclic ring, preferably a saturated heterocyclic ring such as pyrrolidine, piperidine or morpholine. $NR_2R_3$ is preferably a pyrrolidino group or a dimethylamino group.

Suitably $R_4$ is hydrogen, halogen, $C_{1-4}$ alkyl, $C_{1-4}$ alkoxy or trifluoromethyl. Most suitably $R_4$ is hydrogen, methyl, ethyl, trifluoromethyl, methoxy, bromo, chloro or fluoro. Preferably $R_4$ is methyl trifluoromethyl, methoxy, bromo or chloro. Most preferably $R_4$ is methyl.

Preferably X is —N=

A preferred group of compounds of the formula (I) is that of the formula (IV):

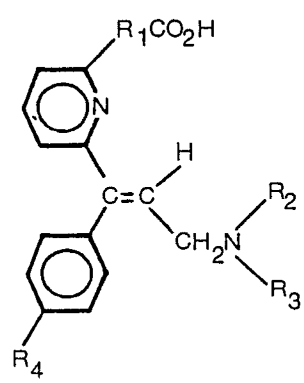

(IV)

or a salt, ester or amide thereof;

wherein $R_1$ to $R_4$ are as hereinbefore defined. Of the compounds of the formula (IV), those wherein $R_1$ is a single bond (i.e. n=O), CH=CH or $CH_2CH_2$, $NR_2R_3$ is pyrrolidino and $R_4$ is methyl or trifluoromethyl are particularly preferred.

A further preferred group of compounds of the formula (I) is that of the formula (V)

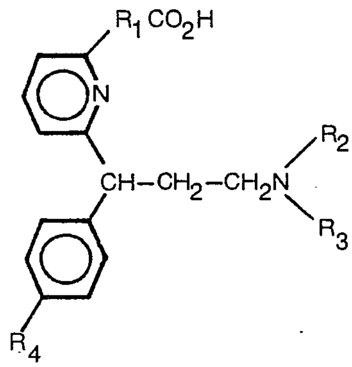

(V)

or a salt, ester or amide thereof; wherein $R_1$ to $R_4$ are as hereinbefore defined. Of the compounds of the formula (V), those wherein $R_1$ is a single bond, CH=CH or $CH_2CH_2$, $NR_2R_3$ is dimethylamino and $R_4$ is chlorine or bromine are particularly preferred.

Amides of the compounds of the formula (I) included within the scope of the invention are amides conventionally formed from carboxylic acids. Amides formed from ammonia, primary amines or amino acid, such as glycine, are particularly suitable.

Solvates of the compounds of the formula (I) are also included within the scope of the present invention. Preferred solvates include hydrates and $C_{1-4}$ alkanolates.

When the compounds of formula (I) contain a double bond in the side chain terminating in the group $NR_2R_3$, for example the compounds of formula (IV), they exist in either the *cis* or *trans* isomeric form(s) (in relation to the X-containing ring). The compounds of the formula (IV) have been drawn in the *trans* configuration and these are the isomers which primarily have useful antihistamine activity. The compounds in the *cis* configuration are primarily useful as intermediates in preparing the *trans* isomers. The present invention also provides mixtures of the isomers. When $R_1$ in the substituent $R_1CO_2H$ contains a double bond, further isomers of the compounds of the formula (I) exist, and both isomers and the isomeric mixture of these compounds are included within the scope of the present invention. When $R_1CO_2H$ contains a double bond, the preferred isomers are those wherein the carboxylic acid group is trans to the aromatic ring.

Esters and amides of the compounds of the formula (I) whilst having some antihistamine activity in their own right may also be useful intermediates in the preparation of the carboxy compounds of the formula (I). Suitable ester includes conventional ester groups known to be useful for protecting carboxylic acid groups such as $C_{1-6}$ alkyl esters wherein the alkyl group is straight or branched chain and is optionally substituted by halogen. Alkyl esters ($C_{1-4}$) are particularly preferred.

Salts of the compounds of formula (I) may be either acid addition salts or salts formed with the carboxylic acid group. Acid addition salts are preferred but salts formed from the carboxylic acid group may be particularly useful in preparing the corresponding carboxy compound. Pharmaceutically acceptable salts are preferred.

When used in medicine, the salts of the compound of formula (I) should be both pharmacologically and pharmaceutically acceptable, but non pharmaceutically acceptable salts may conveniently be used to prepare the free active compound or pharmaceutically acceptable salts thereof and are not excluded from the scope of this invention. Such pharmacologically and pharmceutically acceptable acid addition salts include, but are not limited to, those prepared from the following acids: hydrochloric, sulphuric, nitric, phosphoric, maleic, salicylic, toluene-p-sulphonic, tartaric, citric, methanesulphonic, formic, malonic, isothionic, succinic, naphthalene-2-sulphonic and benzenesulphonic. Also, pharmaceutically acceptable salts can be prepared as alkaline metal or alkaline earth salts, such as sodium, potassium or calcium salts of the carboxylic acid group.

Preferred compounds of the formula (I) include:—

*E*-3-(6-(3-pyrrolidino-1-(4-tolyl)prop-1*E*-enyl)-2-pyridyl)acrylic acid
3-(6-(3-pyrrolidino-1-(4-tolyl)prop-1*E*-enyl)-2-pyridyl)propionic acid
(*E*)-3-(6-(3-dimethylamino-1-(4-tolyl)prop-1*E*-enyl)-2-pyridyl)acrylic acid
(*E*)-3-(6-(3-pyrrolidino-1-(4-trifluoromethylphenyl)prop-1*E*-enyl)-2-pyridyl)acrylic acid
(*E*)-3-(6-(3-pyrrolidino-1-(4-methoxyphgenyl)prop-1*E*-enyl)-2-pyridyl)acrylic acid
(*E*)-3-(6-(1-phenyl-3-pyrrolidinoprop-1-*E*-enyl)-2-pyridyl)acrylic acid
(*E*)-3-(6-(1-(4-chlorophenyl)-3-pyrrolidinoprop-1*E*-enyl)-2-pyridyl)acrylic acid
6-(3-pyrrolidino-1-(4-tolyl)prop-1*E*-enyl)-pyridine-2-carboxylic acid
(*E*)-3-(3-pyrrolidino-1-(4-tolyl)prop-1-enyl)benzoic acid
(*E*)-3-(3-pyrrolidino-1-(4-tolyl)prop-1*E*-enyl)cinnamic acid
(*E*)-3-((*E*)-3-pyrrolidino-1-(4-methoxyphenyl)prop-1-enyl)cinnamic acid
(*E*)-3-((*E*)-3-dimethylamino-1-(4-tolyl)prop-1-enyl)cinnamic acid
(*E*)-3-(3-(3-pyrrolidino-1-(4-tolyl)prop-1-enyl)phenyl)propionic acid

or salts, esters or amides thereof.

Pharmacokinetic studies comparing the relative distribution in brain and plasma or one of the compounds of this invention and triprolidine indicate that, unlike triprolidine, this compound (compound A, see examples) does not readily penetrate the brains of rodents.

The present invention also provides a method for preparing compounds of the formula (I), which method comprises:—

a) The reaction of compound of the formula (VI)

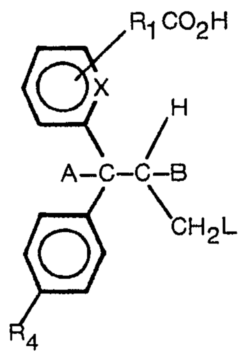

(VI)

or an ester thereof with an amine $HNR_2R_3$ wherein X, A, B and $R_1$ to $R_4$ are as hereinbefore defined and L is a leaving group;

b) When it is required to prepare a compound of the formula (I) wherein $R_1$ is $(CH_2)_0$ and A and B are hyydrogen, the reaction of a compound of the formula (VII)

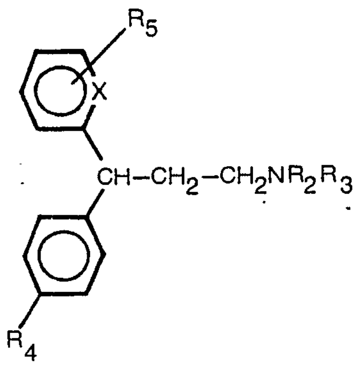

(VII)

wherein $R_2$, $R_3$, $R_4$, X, A and B are as hereinbefore defined and $R_5$ is a halogen atom, with a $C_{1-6}$ alkyl lithium compound followed by treatment with carbon dioxide;

c) When it is required to prepare a compound of the formula (I) wherein $R_1$ is $(CH_2)_a$ $CH{=}CH(CH_2)_b$ and a is 0, the reaction of a compound of the formula (VIII):

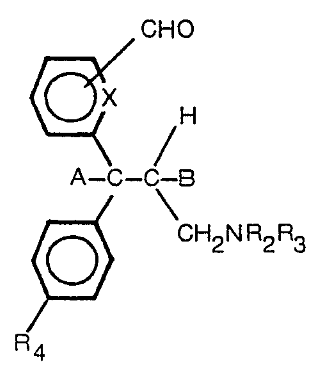

(VIII)

wherein $R_2$, $R_3$, $R_4$, A and B are as hereinbefore defined, with a Wittig reagent suitable for attaching the side chain $CH{=}CH(CH_2)_b$ $COR_6$, wherein $COR_6$ is an acid, ester or amide group as hereinbefore defined, followed by deprotection of the carboxy group if desired;

d) When it is required to prepare a compound of the formula (I) wherein CA—CB represents a double bond:

1) The reaction of an ester, amide or carboxylic acid salt of a compound of the formula (IX):

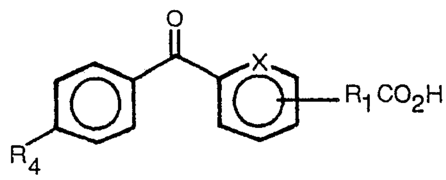

(IX)

with a Wittig reagent suitable for attaching the side chain $=CHCH_2NR_2R_3$ wherein, X and $R_1$ to $R_4$ are as hereinbefore defined, followed by deprotection of the carboxy group if desired;

2) The elimination of $R_7$ OH from a compound of the formula (X):

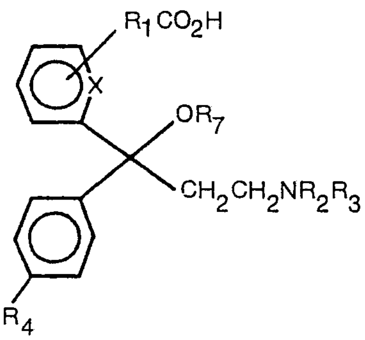

(X)

or an ester or amide thereof, wherein X, $R_1$ to $R_4$ are as hereinbefore defined and $R_7$ is hydrogen or $C_{1-4}$ acyl;

3) The reaction of a compound of the formula (XI):

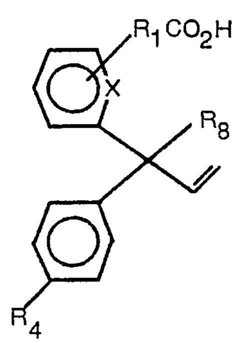

(XI)

with an amine $HNR_2R_3$, wherein $R_1$ to $R_4$ are as hereinbefore defined and $R_8$ is a $C_{1-4}$ acyloxy group;

e) and thereafter, optionally converting one compound of the formula (I) to another compound of the formula (I) by methods well known to those skilled in the art, for example the isomerisation of a compound of the formula (XII)

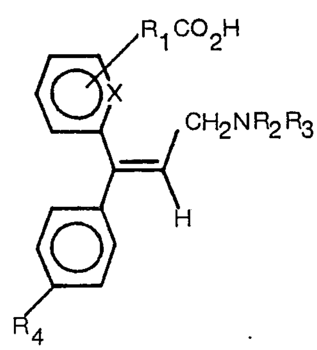

(XII)

when CA—CB is a double bond, the reduction of one or more double bonds or de-esterification of the ester group.

a) Suitable leaving groups L in the compounds of the formula (VI) are those as defined by J. March, *Advanced Organic Chemistry,* 2nd ed., pages 683 and 895, McGraw Hill, New York, 1977, *e.g.* —BR, —Cl, toluene sulphonate, methane sulphonate, acyloxy (such as acetate), etc.

This reaction will normally be carried out in a solvent suitable for carrying out such displacement reactions, for example a polar solvent, such as a $C_{1-4}$ alkanol or a polar aprotic solvent such as DMSO, at a temperature between 0 and 180°C.

The compounds of the formula (VI) may be prepared by the reaction of an ester of the corresponding compound where L is a hydroxy group with an acid or a suitable reactive acid derivative, followed by removal of the ester function if desired. Suitable reactants include hydrogen halides, halogenated phosphorus compounds such as phosphorus pentachloride or phosphorus oxychloride, a suitable sulphonyl chloride (such as methane sulphonyl chloride or p-toluene sulphonyl chloride) or an acid anhydride, such as acetic anhydride. The reaction will conveniently be carried out in a suitbale solvent under conditions well known to those skilled in the art, for example a non-protic solvent such as an ether or a halogenated hydrocarbon, in the present of a base such as a tertiary amine (for example triethylamine) at a non-extreme temperature, for example between 0° and 100°C and conveniently at room temperature. When a tertiary amine is used as a base, an excess of this may be used as the solvent.

The hydroxy compounds may be prepared by the reaction of a compound of formula (IX) with an appropriate Wittig reagent containing a protected hydroxy group for example

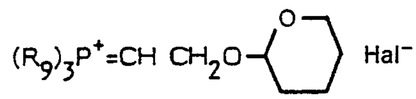

wherein $R_9$ is a $C_{1-4}$ alkyl or phenyl group, which is liberated by the action of strong base on the corresponding phosphonium salt

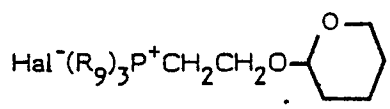

where Hal is chlorine or bromine.

The compounds of the formula (VI) may also be prepared by the rearrangement of a compound of the formula (XI). This rearrangement is suitably carried out in the presence of a catalyst, for example a suitable solubilised palladium catalyst, such as bis-(benzonitrile)palladium (II) dichloride or bis-(acetonitrile)-palladium (II) dichloride, in a suitable solvent, in a suitable polar aprotic solvent, such as acetonitrile, at a non-extreme temperature, for example between 20° and 120°C, most suitably between 40 and 90°C.

b) The reaction of a compound of the formula (VII) with alkyl lithium followed by treatmet with carbon dioxide is suitably carried out in a solvent inert under the reaction conditions utilised, for example benzene, toluene or an ether such as tetrahydrofuran, under an inert atmosphere, such as nitrogen, and at a low temperature, for example between −80°C and −20°C. The alkyl lithium compound is suitably butyl lithium. The reaction is conveniently carried out in toluene or tetrahydrofuran at a temperature between −80°C and −50°C under nitrogen. The compound of the formula (VII) may be prepared by the reduction of a compound of the formula (VIII):

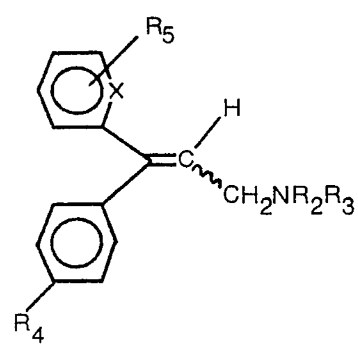

(XIII)

wherein $R_2$ to $R_5$ are as hereinbefore defined, under conditions that will not affect the group $R_5$.

This reduction is suitably carried out by hydrogenation in the presence of a transition metal catalyst, such as platinum on charcoal. The compounds of the formula (XIII) may be conveniently prepared by the reaction of an appropriate Wittig reagent with a compound of the formula (VIX):

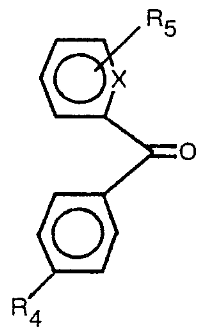

- (XIV)

Analogous Wittig reactions are described elsewhere in this document and the Wittig reagent is suitably a compound of the formula $(R_9)_3\,P{=}CHCH_2NR_2R_3$ which is liberated from the corresponding phosphonium salt $(R_9)_3\,P^+CH_2CH_2NR_2R_3\ Hal^-$ where $Hal^-$ is $Cl^-$ or $Br^-$ by the action of strong base. $R_2$ and $R_3$ are as hereinbefore defined and $R_9$ is a $C_{1-4}$ alkyl or phenyl group. Suitable strong bases are $C_{1-4}$ alkyl or aryl lithium compounds, such as butyl lithium, or metal hydrides, such as sodium hydride. The reaction is suitably carried out in an inert solution, for example an ether such as tetrahydrofuran, at a non-extreme temperature, for example 0° to 50°C and conveniently at room temperature.

(c and d(1)) These reactions are conventional Wittig reactions and, as such, are analogous to those described in *Organic Reactions, 14,* 270—490 (1965) and *Pure and Applied Chemistry, 9,* 245—254 (1964). The reactions are suitably carried out in an anhydrous solvent inert under the reaction conditions utilised, for example toluene, benzene, tetrahydrofuran, dioxan, glycol ethers and $C_{1-6}$ alkyl ethers such as ethyl ether, at a temperature between −80°C and 100°C. The Wittig reagent will normally be prepared by treatment of a phsophonium salt with a strong base, for example a $C_{1-4}$ alkyl or aryl lithium compound such as butyl lithium, or a metal hydride, such as sodium hydride in a suitable inert solvent, such as those specified above.

The Wittig reagent in reaction (c) is conveniently prepared by reacting a compound of the formula $(R_{10})_2\,PO.(CH_2)_d\,CO_2C_6$, wherein $R_6$ is as hereinbefore defined, $R_{10}$ is a $C_{1-4}$ alkoxy group and d is 1 to 6, or a compound of the formula $(R_9)_3P(CH_2)_dCO_2R_6$ wherein $R_9$ and $R_6$ are as hereinbefore defined and d is 1 to 6 with a strong base, such as sodium hydride in a suitable inert solvent, such as tetrahydrofuran or dimethoxyethane at a temperature between 0° and 50°C, conveniently at room temperature.

The reaction between the Wittig reagent and compound of the formula (VIII) is conveniently carried out by adding the compound of the formula (VIII) to the Wittig reagent at a temperature of between 0° and 50°C and conveniently at room temperature.

The compound of formula (VIII) is suitably prepared by oxidation of the corresponding alcohol, for example by oxidation with barium manganate in a halogenated alkane, such as dichloromethane at a non-extreme temperature, for example between 0° and 75°C. The alcohol may be prepared by reduction of the corresponding acid or its ester, i.e. a compound of the formula (I) wherein $R_1$ is $(CH_2)_0$. This reduction may suitably be carried out using a metal hydride, such as lithium aluminum hydride, in an inert solvent, such as an ether, for example diethyl ether, at between 0 and 75°C and suitably under reflux.

The Wittig reagent in direction (d(i)) is conveniently a compound of the formula $(R_9)_3P{=}CHCH_2NR^2R^3$ which can be liberated from its corresponding phosphonium salt $(R_9)_3P^+CH_2CH_2NR_2R_3\ Hal^-$ wherein Hal, $R_2$ and $R_3$ are as hereinbefore defined and $R_9$ is a $C_{1-4}$ alkyl or phenyl group by reaction with a strong base. The reaction is suitably carried out in an inert solvent such as toluene or tetrahydrofuran at a temperature of between 0° and 50°C and conveniently at room temperature. Suitably the strong base is an alkyl or aryl lithium compound, such as butyl lithium, or a metal hydride, such as sodium hydride. The use of butyl lithium in toluene at room temperature has been found to be particularly convenient. The phosphonium salts $(R_9)_3P^+CH_2CH_2NR_2R_3\ Hal^-$ may be prepared by known methods (see, for example, UK Patent No. 1161201).

Compounds of formula (IX) in which $R_1$ is —CH=CH— (trans) may be prepared by reacting a compound of formula (XV) with an acrylate ester (XVI) in the presence of a catalyst consisting of palladium acetate and a triarylphosphine and a tertiary amine such as triethylamine or tributylamine at an elevated temperature, for example 120° to 180°C, conveniently 140° to 150°. The reaction may be carried out under pressure to achieve the desired temperature range if desired. Optionally a solvent such as acetonitrile may be used and the reactants may be heated together in a sealed pressure vessel (e.g. see R. F. Heck *et al., J. Org. Chem., 43,* 2947 (1978)).

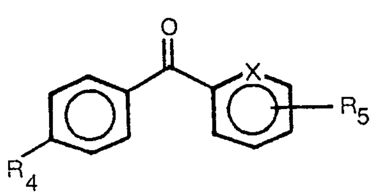

(XV) $CH_2{=}CHCO_2R_9$ (XVI)

wherein $R_4$ and $R_5$ are as defined above and $R_9$ is a $C_{1-4}$ alkyl group.

Compounds of formula (IX) may also be prepared by reacting a compound of formula (XVII):

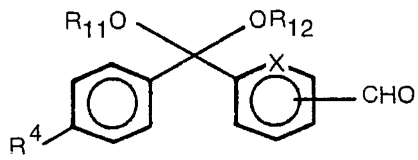

(XVII)

wherein $R_{11}$ and $R_{12}$ may be the same or different and are each $C_{1-4}$ alkyl, or may together form a cyclic ketal containing up to 6 carbon atoms, with malonic acid in the presence of a suitable base such as pyridine or piperidine, or with a Wittig reagent prepared by treating a phosphonium salt (XVIII A) or a phosphonate ester (XVIII B) with a suitable base in an appropriate solvent:

$(R_9)_3P^+(CH_2)_dCO_2R_6Hal^-$ (XVIII A)

$(R_{10})_2PO(CH_2)_dCO_2R_6$ (XVIII B)

wherein Hal, $R_6$, $R_9$, $R_{10}$ and d are as defined above. The ketone (IX) is generated by acidic hydrolysis of the protecting ketal. The double bond in the group $R_1$ may be reduced if desired with hydrogen in presence of a catalyst such as palladium charcoal.

Compounds of formula (XVII) may be prepared from compounds of formula (XV) by conversion to a ketal by reaction with a mono or dihydroxy compound in the presence of an acid catalyst followed by rection with a metal alkyl compound, for example butyllithium, and subsequent treatment with dimethylformamide. The reaction is preferably conducted at low temperature (below −60°C) in a solvent such as toluene.

In turn compounds of formula (XV) can be prepared by treatment of a compound of formula (XVIII) with a metal alkyl compound, for example butyllithium, in a suitable solvent such as toluene, followed by reaction with a compound of formula (XIX) wherein $R_5$ is halogen such as chlorine or bromine and $R_4$ is as hereinbefore defined.

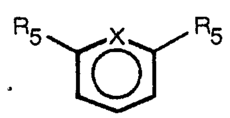

(XVIII)

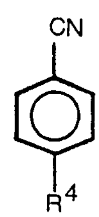

(IXX)

(d(2)) The elimination of $R_7$ OH from compounds of formula (X) is conveniently accomplished in the presence of a strong mineral acid, for eample concentrated sulphuric acid, at an elevated temperature, for example between 100° and 200°C, suitably 125° to 150°C.

The compounds of formula (X) may be prepared by the reaction of a compound of the formula (XX):

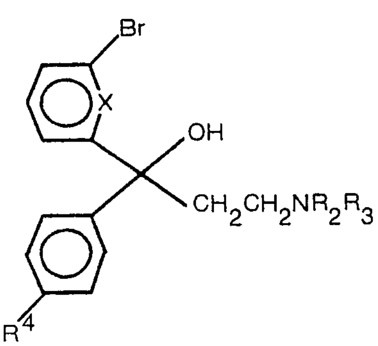

(XX)

with CH=CHCOR$_{13}$ wherein X and $R^2$ to $R^4$ are as hereinbefore defined and COR$_{13}$ is an ester or amide group. This reaction is conveniently carried out in the presence of a catalyst consisting of palladium acetate and a triaryl phosphine and in the presence of a tertiary amine, conveniently a water soluble tertiary amine such as N-ethylmorpholine. The compound of the formula (XX) is conveniently prepared from the reaction of a compound of formula (XVIII) with a metal alkyl compound such as butyl lithium followed by reaction with a compound of the formula (XXI):

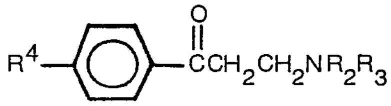

(XXI)

This reaction is suitably carried out at low temperature, for example between −90° and −30°C, conveniently between −70° and −40°C, in an inert solvent, for example toluene, and in an inert atmosphere.

The compounds of the formula (X) may also be prepared by the reaction of a compound of the formula (XXII):

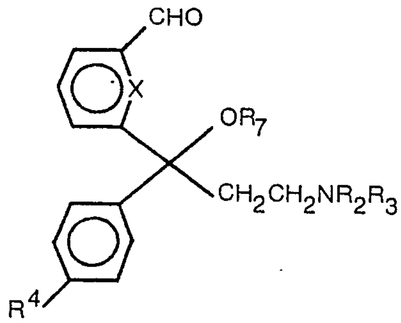

(XXII)

with malonic acid. The reaction is conveniently carried out in pyridine in the presence of a base, for example piperidine, at an elevated temperature, for example between 50° and 100°C. The compounds of the formula (XXII) may be prepared by the reaction of 2-bromo-6-(1,3-dioxolan-2-yl)pyridine or 1-bromo-3-(1,3-dioxolan-2-yl)benzene with a compound of the formula (XXI) as hereinbefore defined followed by acylation if desired.

For example, 2-bromo-6-(1,3-dioxolan-2-yl)pyridine is conveniently mixed with butyl lithium in an inert solvent, such as toluene, at a low temperature, for example between −80° and −40°C, conveniently between −60° and −70°C, in an inert atmosphere, such as nitrogen, before the addition of the compound of the formula (XXI). The reaction is conveniently carried out in an inert solvent, such as toluene, at a low temperature, for example between −80° and −40°C and suitably between −70° and −60°C in an inert atmosphere, conveniently nitrogen.

(d(3)) The reaction of a compound of formula (XI) with an amine $HNR_2R_3$ is suitably carried out in the presence of a palladium catalyst. The reaction is conveniently carried out in a polar aprotic solvent, such as acetonitrile, at an elevated temperature, for example between 20° and 100°C, suitably between 30° and 80°C and conveniently between 50° and 70°C. This reaction is conveniently carried out on an ester of a compound of the formula (XI).

The compounds of formula (XI) may conveniently be prepared by the acylation of the corresponding compound wherein $R_8$ is a hydroxy group. This reaction is suitably carried out by the use of the appropriate acyl anhydride in the presence of base, for example triethylamine. The use of 4-N,N-dimethylaminopyridine as a catalyst has been found to facilitate this reaction. The preparation of the hydroxy compounds is suitably carried out by the reaction of a compound of the formula (IX) with a Grignard reagent $CH_2{=}CHMg$ Hal wherein Hal is a suitable halogen atom such as bromine. this reaction is carried out under conditions conveniently used for Grignard reactions, for example in an inert anhydrous solvent such as tetrahydrofuran and can advantageously be carried out in the presence of zinc chloride thereby generating divinyl zinc which reacts with the compound of the formula (IX) *in situ*.

e) The isomerization of a compound of the formula (XII) is suitably carried out in the presence of in excess of one molar equivalent of a strong acid, suitably a strong mineral acid, for example sulphuric acid, at an elevated temperature, for example between 50° and 160°C, conveniently between 125° and 150°C.

The compounds of the formula (XII) may be prepared as by-products in some of the reaction methods for the preparation of compounds of the formula (I) and may be obtained from the reaction mixture by conventional separation techniques, for example by chromatography or by techniques that rely on solubility differences between the two isomers in a suitable solvent, for example, it has been found that when it is required to prepare a compound of the formula (IV) as the free acid, it is often convenient to prepare the corresponding ester and then saponify this, for example with an alkali metal hydroxide, such as sodium hydroxide, in a $C_{1-4}$ alkanol, such as ethanol, to give the acid.

The reduction of one or two double bonds, i.e. the reduction of the double bond terminating in the group $NR_2R_3$ or the reduction of its double bond in the carboxy side chain may conveniently be carried out by hydrogenation in the presence of a transition metal catalyst, for example platinum on charcoal. The preparation of esters or amides from the corresponding carboxylic acid, and vice versa, may similalry be carried out by methods well known to those skilled in the art.

Those intermediates of the formulae (X) to (XII) form an important further aspect of the present invention.

The compounds of this invention may be used for the same indications as triprolidine, namely to relieve symptoms of nasal stuffiness due to colds and vasomotor rhinitis and for the symptomatic control of allergic conditions including nasal allergy, perennial rhinitis, urticaria, angioneurotic oedema, allergic conjunctivitis, food allergy, drug and derum reactions, insect bits and stings and desensitizing reactions. The compound may also be used in conditions responsive to its antipruritic activity including allergic dermatoses, neurodermatitis, anogenital pruritus, and pruritus of non-specific origin such as eczema, and of specific cause such as chickenpox, photosensitivity and sunburn. Some of the compounds of the present invention have been found to be substantially free from sedative effects and to have little or no anticholinergic effects.

The amount of active compound required for use in the above conditions will vary with the compound chosen, the route of administration and the condition and mammal undergoing treatment, and is ultimately at the discretion of the physician. A suitable oral dose of the active compound for a mammal is in the range of from 0.003 to 1.0 mg per kilogram body weight per day; preferably from 0.04 to 0.24 mg/kg. For example a typical dose for a human recipient of compound (A) (see example 1 and Table 1 hereafter) is 0.12 mg/kg body weight per day.

The desired daily dose is preferably presented as from one to six sub-doses administered at appropriate intervals throughout the day as needed. Where three subdoses of compounds of formula (I) are employed, each will preferably lie in the range of from 0.014 to 0.08 mg/kg body weight; for example, a typical sub-dose of such a compound for a human recipient is between 1 and 20 mg, for example 4 or 8 mg.

Whilst it is possible for a compound of the formula (I) to be administered alone as the raw chemical, it is preferable to present the compound of formula (I) as a pharmaceutical formulation. Thus, the present invention also provides pharmaceutical formulations, both for veterinary and for human medical use, which comprise a compound of the formula (I) together with one or more pharmaceutically acceptable carriers therefore and optionally any other therapeutic ingredients. For example, the active compound may be formulated with a sympathomimetic agent such as the decongestant pseudoephedrine, an antitussive such as codeine, an analgesic, an antiinflammatory, an antipyretic, or an expectorant. The carrier(s) must be pharmaceutically acceptable in the sense of being compatible with the other ingredients of the formulation and not deleterious to the recipient thereof.

The formulations include those suitable for oral, rectal, topical, nasal, ophthalmic or parenteral (including subcutaneous, intramuscular and intravenous) administration.

The formulations may conveniently be presented in unit dosage form and may be prepared by any of the methods well known in the art of pharmacy. All methods include the step of bringing the active compound into association with a carrier which constitutes one or more accessory ingredients. In general, the formulations are prepared by uniformly and intimately briniging the active compound into association with a liquid carrier or a finely divided solid carrier or both and then, if necessary, shaping the product into desired formulations.

Formulations of the present invention suitable for oral administration may be presented as discrete units such as capsules, cachets, tablets or lozenges, each containing a predetermined amount of the active compound (defined herein as a compound of formula (I)); as a powder or granules; or a suspension in an aqueous liquid or nonaqueous liquid such as a syrup, and elixir, an emulsion or a draught.

A tablet may be made by compression or molding, optionally with one or more accessory ingredients. Compressed tablets may be prepared by compressing in a suitable machine, with the active compound being in a free-flowing form such as a powder or granules which is optionally mixed with a binder, disintegrant, lubricant, inert diluent, surface active agent or dispersing agent. Molded tablets comprised of a mixture of the powdered active compound with any suitable carrier may be made by molding in a suitable machine.

A syrup may be made by adding the active compound to a concentrated, aqueous solution of a sugar for example sucrose to which may also be added any accessory ingredient(s). Such asccessory ingredient(s) may include flavourings, an agent to retard crystallization of the sugar or an agent to increase the solubility of any other ingredient, such as a polyhydric alcohol, for example glycerol or sorbitol, and suitable preservatives.

Formulations for rectal administration may be presented as a suppository with a usual carrrier such as cocoa butter, or hydrogenated fats or hydrogenated fatty carboxylic acids.

Formulations suitable for parenteral administration conveniently comprise a sterile aqueous preparation of the active compound which is preferably isotonic with the blood of the recipient.

Nasal spray formulations comprise purified aqueous solutions of the active compound with preservative agents and isotonic agents. Such formulations are adjusted to a pH and isotonic state compatible with the nasal mucous membranes.

Ophthalmic formulations are prepared by a similar method to the nasal spray except that pH and isotonic factors are adjusted to match that of the eye.

Topical formulations comprise the active compound dissolved or suspended in one or more media such as mineral oil, petroleum, polyhydroxy alcohols or other bases used for topical pharmaceutical formulations. The addition of other accessory ingredients, *vide infra,* may be desirable.

In addition to the aforementioned ingredients, the formulations of this invention may further include one or more accessory ingredient(s) selected from diluents, buffers, flavouring agents, binders,

disintegrants, surface active agents, thickeners, lubricants, preservatives (including antioxidants) and the like.

The present invention also provides the first use of the compounds of the formula (I) in medicine.

The following Examples are provided by the way of illustration of the present invention and should in no way be construed as a limitation thereof. All temperatures indicated are in degrees Celcius.

Example 1

(E)-3-(6-(3-Pyrrolidino-1-(4-tolyl)prop-1E-enyl)-2-pyridyl)acrylic acid (Compound A).

Butyllithium (50 mL, 1.65M in hexane) was added under nitrogen to a stirred suspension of 2,6-dibromopyridine (19.5 g) in dry ether (200 mL) at −50°. After 0.75 hr a solution of 4-tolunitrile (10 g) in ether (50 mL) was added; stirring was continued at −50° for 3 hrs. The mixture was allowed to warm to −30° and treated with hydrochloric acid (200 mL, 2M). The precipitated solid was collected, washed with water, and recrystallized from aqueous ethanol. The 2-bromo-6-(4-toluoyl)pyridine formed colorless needles (12.2 g) m.p. 97—98°.

A mixture of 2-bromo-6-(4-toluoyl)pyridine (200 g), ethylene glycol (85 mL), *p*-toluenesulphonic acid (32 g) and benzene (11 mL) was boiled under a Dean/Stark trap until water collection had become very slow (about 20 mL collected in 16 hours).

The cooling solution was poured into ice/water containing sodium carbonate (100 g) with stirring. The benzene layer was separated, washed with water, dried with sodium sulphate and evaporated to about 500 mL. Cooling gave a first crop of 2-(6-bromo-2-pyridyl)-2-(4-tolyl)-1,3-dioxolan (compound 1), m.p. 113—114° (170 g). Dilution with petroleum ether gave a second crop, m.p. 109—112° (34 g). The residue after evaporation (31 g) was recycled.

A solution of compound 1, *vide supra,* (70 g) in dry toluene (800 mL) was added dropwise during 5 hr to a stirred solution of butyllithium (1.6M in hexane, 200 mL) and toluene (200 mL) at −65 to −72° under nitrogen. After a further 30 minutes at −70°, dry dimethylformamide (40 mL) was added during 35 minutes. Stirring continued overnight at −70 to −60°.

Hydrochloric acid (2N, 400 mL) was added, allowing the temperature to rise to about −10°. After 30 minutes, 2N ammonia (*ca.* 90 mL) was added to pH 7—8. The toluene layer was separated and the aqueous phase was extracted with ether. The combined organic liquids were washed with ice/water, dried ($MgSO_4$) and evaporated *in vacuo* below 50°. The aldehyde, 2-(6-formyl-2-pyridyl)-2-(4-tolyl)-1,3-dioxolan, (63.9 g) crystallized on keeping at 3°, m.p. 52—63°.

The aldehyde prepared above (2.5 g) was dissolved in 1,2-dimethoxyethane (10 mL) and added to a solution of the phosphonate carbanion produced from triethyl phosphonoacetate (2 g) and sodium hydride (0.22 g) in the same solvent. The mixture was stirred for two hours, diluted with ether (25 mL) and treated with hydrochloric acid (5 mL, 2M). The organic phase was separated, washed with water, dried, and evaporated. The resulting oil was dissolved in ethanol (20 mL) containing concentrated hydrochloric acid (3 mL) and water (3 mL). After heating of the steam bath for ten minutes, the solution was diluted with ice water, rendered alkaline with sodium bicarbonate solution, and extracted with ether. Evaporation gave (*E*)-3-(6-(4-toluoyl)-2-pyridyl)acrylate (compound 2) which crystallized from cyclohexane in colourless platelets (1 g), m.p. 108—111°.

Butyllithium (10 mL, 1.64M in hexane) was added under nitrogen to a stirred suspension of triphenyl-2-pyrrolidonoethylphosphonium bromide (7.2 g) in dry toluene (75 mL). After 0.5 hr, compound 2, *vide supra,* (4.8 g) in toluene (50 mL) was added. The suspension, initially orange, became deep purple, then slowly faded to yellow during 2 hours heating at 75°. The cooled solution was diluted with ether (150 mL) and treated with hydrochloric acid (50 mL, 2M). The aqueous phase was separated, washed with ether, and basified with potassium carbonate (ice) and extracted with ether. The mixture of isomeric esters obtained by evaporation was dissolved in ethanol (100 mL) containing sodium hydroxide solution (20 mL, 1M) and partially evaporated on the steam bath under reduced pressure for 5 minutes. The residual aqueous solution was neutralized with sulphuric acid (20 mL, 0.5M) and evaporated to dryness. The solid residue was extracted with hot isopropanol (3 × 50 mL) and the extracts were concentrated until crystallization commenced. The (*E*)-2-(6-(3-pyrrolidino-1-(4-tolyl)prop-1*E*-enyl)-2-pyridyl)acrylic acid (Compound A), after recrystallization from isopropanol, melted at 222° (decomp).

Example 2

3-(6-(3-Pyrrolidino-1-(4-tolyl)prop-1E-enyl)-2-pyridyl)propionic acid (Compound B).

A solution of compound 2, *vide supra,* (3 g) in alcohol (100 mL) containing Raney nickel (1 g) was stirred under hydrogen at room temperature and pressure until the calculated quantity of hydrogen had been absorbed (*ca.* 45 minutes). The reduced ester was recovered by filtration and evaporation and purified by column chromatography on silica gel using petroleum ether as eluent. Treatment of this ester with Wittig reagent by the method of Example 1 followed by saponification gave a mixture of two isomeric acids which were separated by fractional crystallization from ethyl acetate/petroleum ether mixtures. The less soluble E isomer, 3-(6-(3-pyrrolidino-1-(4-tolyl)prop-1*E*-enyl)-2-pyridyl)propionic acid (Compound B), melted at 156—7°.

Example 3

(E)-3-(6-(3-Dimethylamino-1-(4-tolyl)prop-1E-enyl)-2-pyridyl)acrylic acid (Compound C).

Treatment of compound 2, *vide supra,* with the Wittig reagent derived from triphenyldimethylaminoethylphosphonium bromide by the method of Eample 1 gave a mixture of isomeric acids which were separated by fractional crystallization from ethyl acetate. The less soluble E-isomer, (*E*)-3-(6-(3-dimethylamino-1-(4-tolyl)prop-1*E*-enyl)-2-pyridyl)acrylic acid (Compound C), was purified by crystallization from isopropanol, m.p. 222—5° (decomp.)

Example 4

(E)-3-(6-(3-Pyrrolidino-1-(4-tolyl)prop-1E-enyl)-2-pyridyl)acrylic acid

A mixture of 2-bromo-6-(4-toluoyl)pyridine (56 g), ethyl acrylate (25 mL), triethylamine (30 mL), palladium (II) acetate (0.4 g), triphenylphosphine (0.9 g) and acetonitrile (50 mL) was placed in an autoclave and heated with stirring at 150° for six hours. After cooling the solid product was broken up, washed with water and alcohol, and recrystallized from alcohol, yielding compound 2 (51 g) as colourless prisms mp. 110—12°.

Triphenyl-2-pyrrolidinoethylphosphonium bromide (72 g) was suspended in dry toluene (750 mL) under nitrogen, cooled in ice, and treated during 15 minutes with butyllithium (100 mL, 1.6M in hexane). The bath was removed and stirring continued for six hours. Again with ice cooling compound 2, *vide supra,* (48 g) dissolved in dry toluene (500 mL) was added during 30 minutes. The mixture was then heated in a bath at 75° for 2 hours. Next day, with ice cooling hydrochloric acid (500 mL, 2M) was added. The aqueous layer was separated, washed with ether, basified with ammonium hydroxide (ice) and extracted with ether. Drying and evaporation gave a mixture of basic esters (46 g). Concentrated sulphuric acid (75 mL) was added and the mixture was plunged into an oil bath at 150° and stirred for 5 minutes. After rapid cooling the mixture was cautiously added to methanol (500 mL). After boiling under reflux for one hour the solution was evaporated to 200 mL *in vacuo,* poured onto excess ice and basified with ammonium hydroxide. Extraction with ether, drying the washed extracts and evaporation gave a dark oil (39 g). Ethanol (750 mL) and sodium hydroxide solution (150 mL, 1M) were added and the mixture was heated on the steam bath under reduced pressure to remove the alcohols as rapidly as possible. To the remaining aqueous solution sulphuric acid (150 mL, 0.5M) was added and the neutral solution was evaporated to dryness *in vacuo.* The dry solid remaining was extracted with hot isopropanol (4 × 200 mL). Partial evaporation and cooling gave 19 g of (*E*)-3-(6-(3-pyrrolidino-1-(4-tolyl)prop-1*E*-enyl)-2-pyridyl)acrylic acid, m.p. 220—220° (decomp.).

Example 5

(E)-3-(6-(3-Pyrrolidino-1-(4-tolyl)-prop-1E-enyl)-2-pyridyl)acrylic acid

A solution of 2-bromo-6-(1,3-dioxolan-2-yl)pyridine (91 g) in toluene (50 mL) was added under nitrogen to a stirred mixture of butyllithium (260 mL, 1.6M) and toluene (1.1 L) between −60 and −70°. After 2 h a solution of 1-pyrrolidino-3-(4-tolyl)propan-3-one (prepared from 85 g of the corresponding hydrochloride and dried) in toluene (200 mL) was added at −70° and the mixture stirred for a further 3 hours at this temperature. The solution was allowed to warm to −20° and treated with hydrochloric acid (510 ml, 2M). The separated aqueous layer was washed with ether, basified with sodium hydroxide solution (10M) at 0° and extracted with toluene. Evaporation of the dried extracts gave an oil (120 g). This was dissolved in hydrochloric acid (200 ml, 2M) and heated on the steam bath for 30 minutes. Cooling, basification and re-isolation gave 2-(1-hydroxy-3-pyrrolidino-1-(4-tolyl)-propyl)pyridine-6-aldehyde as an oil (115 g). The crude aldehyde was dissolved in pyridine (133 mL) and reacted with malonic acid (58 g) in presence of piperidine (2 mL) at reflux for one hour. After evaporation *in vacuo,* the residue was dissolved in a small volume of glacial acetic acid, diluted with water (2 L) and set aside at 0° to crystallise. The solid product was esterified with methanol and sulphuric acid giving methyl-(*E*)-3-(6-(1-hydroxy-3-pyrrolidino-1-(4-tolyl)propyl-2-pyridyl)acrylate as a dark oil (27 g). A small sample crystallized from petroleum ether in colourless prisms, m.p. 75—77°. A mixture of the crude ester (25 g) and concentrated sulphuric acid (50 ml) was heated in an oil bath at 160° for 20 minutes. Re-isolation and saponification by the method described in Example 5 gave (*E*)-3-(6-(3-pyrrolidino-1-(4-tolyl)prop-1*E*-enyl)-2-pyridyl)acrylic acid as off-white crystals, m.p. 218—9° (decomp.). A further recrystallization from isopropanol raised the melting point to 222—3°.

Example 6

6-(3-Pyrrolidino-1-(4-tolyl)prop-1E-enyl)-pyridine-2-carboxylic acid

A solution of compound I (7 g) in dry toluene (80 ml) was added dropwise under nitrogen to a stirred solution of butyl lithium (1.6M in hexane, 20 ml) cooled below −60°. After three hours at this temperature solid carbon dioxide (25 g) was added. The mixture was allowed to warm to 10°, treated with hydrochloric acid (2M, 20 ml) and filtered from a small quantity of solid (3). The toluene layer was separated and concentrated, leaving an oil (7 g). This was heated on the stream bath for ten minutes with 6M hydrochloric acid (10 ml) containing just sufficient alcohol to give a clear solution. Cooling and dilution with water gave a gummy solid which crystallised from water in colourless needles m.p. 151—3°. (Treatment of the solid 3 with hydrochloric acid afforded a further 0.9 g of the same material). Esterification of this acid with ethanol/sulphuric acid afforded after the usual work up procedure ethyl 6-(4-tolyl)-pyridine-2-carboxylate (Compound 4) (2.8 g) as a colourless oiil which slowly crystallised.

Treatment of compound 4 with the Wittig reagent derived from triphenyl-2-pyrrolidinoethylphosphonium bromide by the method of Example I gave, after saponification, a mixture of two geometrical isomers, which were separated by extraction with hot ethyl acetate. The insoluble E-isomer (the title compound), after crystallisation from isopropanol, melted at 200—202°. Cooling of the ethyl acetate solution from Example 3 led to crystallisation of the more soluble Z isomer, m.p. 187—9°.

Example 7

(E)-3-(6-Pyrrolidino-1-(4-trifluoromethylphenyl)prop-1E-enyl)-2-pyridyl)acrylic acid

2-Bromo-6-(4-trifluoromethylbenzoyl)pyridine, m.p. 66—68° (prepared from 2,6-dibromopyridine and 4-trifluoromethylbenzonitrile by the method of Ex. 1 was converted by the method of Ex. 4 to (*E*)-ethyl-3-[6-(4-trifluoromethylbenzoyl)-2-pyridyl]acrylate, m.p. 129—132°. Further treatment with Wittig reagent by the method of Ex. 1 gave, after saponification and crystallisation from isopropanol, (*E*)-3-(6-(3-pyrrolidino)-(4-trifluoromethylphenyl)prop-1(*E*)-enyl)-2-pyridyl)acrylic acid, m.p. 223—225° (decomp).

Example 8

(E)-3-(6-(3-Pyrrolidino-1-(4-methoxyphenyl)prop-1E-enyl)-2-pyridyl)acrylic acid

4-Methoxybenzonitrile was converted by the method of Ex. 7 to 2-bromo-6-(4-methoxybenzoyl)-pyridine, m.p 116—8°, and thence to (*E*)-ethyl-3-(6-(4-methoxybenzoyl)-2-pyridyl)acrylate m.p. 99—100° and further to (*E*)-3(6-(3-pyrrolidino-1-(4-methoxyphenyl)prop-1*E*-enyl)-2-pyridyl)acrylic acid, which formed colourless crystals from isopropanol, m.p. 231—2° (decomp).

Example 9

(E)-3(6-(1-Phenyl-3-pyrrolidinoprop-1E-enyl)-2-pyridyl)acrylic acid

By the method of Ex. 7 benzonitrile was reacted with 2,6-dibromopyridine to produce 2-bromo-6-benzoylpyridine, m.p. 56—62° which by further processing afforded (*E*)-ethyl 3-(6-benzoyl-2-pyridyl)-acrylate, m.p. 34—36°. Treatment with Wittig reagent then gave (*E*)3-(6-(1-phenyl-3-pyrrolidinoprop-1*E*-enyl)-2-pyridyl)acrylic acid which formed white prisms from ethyl acetate, m.p. 180—182° (decomp).

Example 10

(E)-3-(6-(3-Pyrrolidino-1-(4-tolyl)prop-1E-enyl)-2-pyridyl)acrylamide oxalate

A solution of compound A (1.75 g) (from Example 1) in dry dichloromethane (15 ml) containing N-methylmorpholine (0.31 g) was cooled to −20° and treated with isobutyl chloroformate (0.45 g). After 2 minutes a slow stream of ammonia gas was passed in for 10 minutes. The mixture was stirred at 0° for 1 hour and treated with water (10 ml). The organic phase was separated, washed with water, dried and evaporated to dryness. Treatment of the residual amide (1.4 g) with oxalic acid (0.3 g) in isopropanol gave the title compound as colourless crystals, m.p. 198—9° (decomp).

Example 11

Ethyl (E)-3-(6-(3-Pyrrolidino-1-(4-tolyl)prop-1E-enyl)-2-pyridyl)acrylate oxalate

A solution of compound A (0.5 g) (from Example 1) in ethanol (25 ml) containing sulphuric acid (0.5 ml) was boiled under reflux for 2.5 hours and rapidly evaporated to 10 ml *in vacuo*. The solution was treated with ice and excess ammonia solution and extracted with ether. Addition of oxalic acid (0.13 g) in ethanol (5 ml) to the dried ethereal solution gave a precipitate of the oxalate salt which crystallised from ethyl acetate as white prisms, m.p. 155—6°.

Example 12

(E)-3-(6-(1-(4-Chlorophenyl)-3-pyrrolidinoprop-1E-enyl)-2-pyridyl)acrylic acid

Following the method of Ex. 5, 1-(4-chlorophenyl)-3-pyrrolidinopropan-1-one was converted into the title compound which formed white crystals from isopropanol, m.p. 218—220°.

Example 13

3-(6-(3-Pyrrolidino-1-(4-tolyl)prop-1E-enyl)-2-pyridyl)prop-2E-enamidoacetic acid

A solution of isobutylchloroformate (1.44 g) in dry dichloromethane (5 ml) was added to a stirred and cooled (−25°) solution of Compound A (3.85 g) in dichloromethane (30 ml) containing N-methylmorpholine (1.1 g). After 2 minutes a solution of glycine methyl ester hydrochloride (1.25 g) and N-methylmorpholine (1 g) in dichloromethane (25 ml) was added. The mixture was kept at 0° for one hour, then treated with potassium bicarbonate solution (12 ml, 2 M). The organic phase was separated, washed with water, dried and evaporated. The oily ester so obtained was saponified and the resulting acid was crystallised from aqueous isopropanol. The title compound formed colourless prisms, m.p. 257—8° (decomp).

Example 14

(E)-3-(3-pyrrolidino-1-(4-tolyl)prop-1-enyl)benzoic acid

To a stirred and cooled suspension of triphenyl-2-pyrrolidinoethylphosphonium bromide (17.6 g) in tetrahydrofuran (96 ml) was added a solution of butyl lithium in hexane (28 ml, 1.6*M*) in portions, the temperature being kept at 0°. AFter a further 30 minutes' stirring at 0°, a solution of 3-methoxycarbonyl-4'-methylbenzophenone (Smith, *J. Amer. Chem. Soc.*, 1921, *43*, 1921) (10.16 g) in tetrahydrofuran (50 ml) was

added dropwise, and the mixture was allowed to come to room temperature and then heated at 55° for 18 hours. Most of the tetrahydrofuran was evaporated *in vacuo,* water and dilute hydrochloric acid were added and the mixture was washed with ether. The clear aqueous solution was basified with 2*N*-sodium carbonate solution and the precipitated oil was extracted with ether. Purification by chromatography on a column of silica with a chloroform-methanol (50:1) mixture as eluant gave a mixture of the (*E*)- and (*Z*)-forms of methyl 3-(3-pyrrolidino-1-(4-tolyl)prop-1-enyl)benzoate as a cream-coloured solid (9.8 g).

A solution of the foregoing ester (1.34 g) in ethanol (8 ml) and 2*N*-sodium hydroxide solution (3 ml) was stirred at room temperature for 3 hours. After addition of 2*N*-hydrochloric acid (3 ml) the solution was evaporated to dryness. The residue was extracted with boiling ethanol (2 × 20 ml) to leave an insoluble residue which was washed with water to leave (*Z*)-3-(3-pyrrolidino-1-(4-tolyl)-prop-1-enyl)benzoic acid (300 mg) crystallising from methanol in colourless needles, m.p. 238—240° (decomp) (hydrochloride, m.p. 205—207°). The ethanol extract was evaporated to dryness and the residue was recrystallised from methanol to give colourless prisms (285 mg), m.p. 210—215° (decomp.), of (*E*)-3-(3-pyrrolidino-1-(4-tolyl)prop-1-enyl)benzoic acid (hydrochloride, m.p. 180—182°). Further amounts of the individual isomers were obtained by appropriate recrystallisation of the residue from the filtrate.

Example 15

(E)-3-(3-pyrrolidio-1-(4-tolyl)prop-1-enyl)cinnamic acid

To a stirred suspension of lithium aluminium hydride (330 mg) in ether (62 ml) was added methyl 3-(3-pyrrolidino-1-(4-tolyl)prop-1-enyl)benzoate (mixture of (*E*) and (*Z*)-isomers (Example 14) and the mixture was refluxed for 6 hours. Water (0.33 ml) was added, followed by sodium hydroxide solution (15%, 0.33 ml) and finally water (1 ml), nd the solid was filtered off and washed with ether. The ether filtrate was evaporated to give an oil (4.1 g) which when cooled in solution in a mixture of ether and light petroleum (b.p. 40—60°) deposited crystals 1.43 g); recrystallisation from light petroleum (b.p. 60—80°) gave pure (*E*)-3-(3-pyrrolidino-1-(4-tolyl)prop-1-enyl) benzyl alcohol as colourless needless, m.p. 96—97°. The ether-light petroleum filtrate was evaporated and the residue was separated by high performance liquid chromatography (silica, dichloromethane:methanol:triethylamine 98.5:1.25:0.25) to give more of the above (*E*)-isomer and also (*Z*)-3-(3-pyrrolidino-1-(4-tolyl)-prop-1-enyl)benzyl alcohol which formed colourless prisms, m.p. 67—69°, from light petroleum (b.p. 60—80°).

To a stirred solution of the above (E)-3-(3-pyrrolidino-1-(4-tolyl)-prop-1-enyl)benzyl alcohol (1.1 g) in dichloromethane (75 ml) was added barium manganate (Firouzabadi and Ghaderi, *Tetrahedron Letters,* 1978, 839) and the mixture was kept at 40° for 7 hours, and left at room temperature for 16 hours. The solid was removed by filtration and the filtrate was evaporated to give the crude. (*E*)-3-(3-pyrrolidino-1-(4-tolyl)prop-1-enyl)-benzaldehyde (1.09 g) which was not further purified. To a stirred suspension of sodium hydride (107 mg; 80% oil suspension) in 1,2-dimethoxyethane (3.7 ml) was added diethyl methoxycarbonylmethyl-phosphonate (740 mg) in 1,2-dimethoxyethane (3.7 ml). After 15 minutes' stirring, a solution of the above (*E*)-aldehyde (1.09 g) in 1,2-dimethoxyethane (3.7 ml) was added dropwise and the mixture was stirred for 18 hours at room temperature. After addition of water and acidification with dilute hydrochloric acid, the suspension was washed with ether, and the clear aqueous solution was basified with sodium carbonate solution and the precipitated oil was extracted into ether. The washed and dried ether solution was evaporated to leave a solid (800 mg) which was dissolved in ethanol and refluxed with Girard reagent P (200 mg) for 1 hour. The solvent was evaporated, water and ether were added and the ether extract was washed, dried and evaporated to leave methyl (*E*)-3-((*E*)-3-pyrrolidino-1-(4-tolyl)prop-1-enyl)-cinnamate, m.p 102—107° (620 mg).

This ester (620 mg) was dissolved in ethanol (7 ml), 2*N*-sodium hydroxide solution (2.85 ml) was added and the mixture was stirred for 4 hours at room temperature. 2*N*-Hydrochloric acid (2.85 ml) was added and the solution was evaporated to dryness. Extraction of the solid residue with ethanol, and evaporation of the filtered extract, yielded a solid (600 mg) which was recrystallised from aqueous isopropanol to give light tan-coloured plates, m.p. 190° (decomp.), of (*E*)-3-((*E*)-3-pyrrolidino-1-(4-tolyl)prop-1-enyl)cinnamic acid. The compound formed a hydrochloride, m.p. 240—245° (decomp.).

Example 16

4-(3-Pyrrolidino-1-(4-tolyl)prop-1-enyl)benzoic aid

Under the conditions descriobed in Example 14, 4-methoxycarbonyl-4'-methylbenzophenone (Smith, *J. Amer. Chem. Soc.,* 1921, *43,* 1921) reacted with the phosphorane derived from triphenyl-2-pyrrolidino-ethylphosphonium bromide to give a mixture of the (*E*) and (*Z*)-forms of methyl 4-(3-pyrrolidino-1-(4-tolyl)prop-1-enyl)benzoate. After hydrolysis as described in Example 14, the mixture of acids was easily separated by crystallisation from ethanol to give (*E*)-4-(3-pyrrolidino-1-(4-tolyl)prop-1-enyl)benzoic acid as small colourless prisms, m.p. 235—240° (decomp.) (hydrochloride, m.p. 250° (decomp.)) and (*Z*)-4-(3-pyrrolidino-1-(4-tolyl)prop-1-enyl)benzoic acid as colourless needles, m.p. 245—250° (decomp.) (hydrochloride, m.p. greater than 260° (slow decomp).

Example 17

4-(3-Pyrrolidino-1-(4-tolyl)prop-1-enyl)cinnamic acid

A mixture of 4-bromo-4'-methylbenzophenone (Slootmaekers, Roosen and Verhulst, *Bull. Soc. Chim. Belges.*, 1962, *71*, 446) (6.9 g), ethyl acrylate (2.65 g), palladium (II) acetate (100 mg), triphenylphosphine (225 mg) and triethylamine (2.65 g) in acetonitrile (20 ml) was heated in a stainless steel autoclave under a nitrogen atmosphere at 155° for 5 hours. After cooling, water was added and the precipitated solid was recrystallised from methanol to give colourless plates (2.5 g), m.p. 105.5—106.5°, of ethyl 4-toluoylcinnamate. A further amount of the same material (2.45 g) was isolated by evaporation of the mother-liquor and chromatographic purification of the residue dissolved in dichloromethane on a column of silica.

Reaction of the foregoing ketone (5.9 g) with the phosphorane derived from triphenyl-2-pyrrolidinoethylphosphonium bromide (8.8 g) under the conditions described in Example 14 yielded a crude mixture of the (*E*)- and (*Z*)-forms of ethyl 4-(3-pyrrolidino-1-(4-tolyl)prop-1-enyl)cinnamate (4.3 g).

A solution of the above crude ester mixture in ethanol (43 ml) and 2*N*-sodium hydroxide solution (20 ml) was stirred at room temperature for 4 hours. After neutralisation with 2*N*-hydrochloric acid (20 ml) the solution was evaporated to dryness and the residue was boiled with ethanol. After removal of the insoluble sodium chloride, the filtrate was cooled and deposited crystals (1.1 g) of (*E*)-4-((*E*)-3-pyrrolidino-1-(4-tolyl)prop-1-enyl)cinnamid acid which crystallised from methanol as small colourless prisms, m.p. 225—230° (decomp.) (hydrochloride, m.p. *ca.* 250° (decomp.)). The ethanol mother-liquor was evaporated and the residue was chromatographed on a column of silica in chloroform-methanol (1:1) solution, to give pure (*E*)-4-((*Z*)-3-pyrrolidino-1-(4-tolyl)prop-1-enyl)cinnamic acid (1.1 g) which crystallised from methanol as colourless prismatic needles, m.p. 210—220° (decomp.) (hydrochloride, m.p. 230—235° (decomp.)).

Example 18

3-(3-Pyrrolidino-1-(4-methoxyphenyl)prop-1-enyl)cinnamic acid

Under the conditions described in Example 17, the reaction of 3-bromo-4'-methoxybenzophenone (Allen, Schumann, Day and Van Campen, *J. Amer, Chem. Soc.*, 1958, *80*, 591) (14.6 g) with ethyl acrylate (5.3 g) yielded ethyl 3-(4-methoxybenzoyl)cinnamate, m.p. 71—71.5° (5.6 g).

The foregoing ketone (3.1 g) reacted with the phosphorane derived from triphenyl 2-pyrrolidinoethylphosphonium bromde (4.4 g) under the conditions described in Example 14 to give a crude mixture of the (*E*)- and (*Z*)-forms of ethyl 3-(3-pyrrolidino-1-(4-methoxyphenyl)prop-1-enyl)cinnamate (4.3 g).

This crude ester mixture was hydrolysed with aqueous ethanolic sodium hydroxide solution as described in Example 14 to give the mixed carboxylic acids. The mixture was separated by repeated recrystallisations from ethanol or a mxiture of methanol and ether to give (*E*)-3-((*Z*)-3-pyrrolidino-1-(4-methoxyphenyl)prop-1-enyl)cinnamic acid as small colourless prisms, m.p. 215—220° (decomp.), and (*E*)-3-((*E*)-3-pyrrolidino-1-(4-methoxyphenyl)prop-1-enyl)cinnamic acid as colourless needles, m.p. 220—225° (decomp).

Example 19

3-(3-Pyrrolidino-1-(4-chlorophenyl)prop-1-enyl)cinnamic acid

3-Bromo-4'-chlorbenzophenone (m.p. 118—119°; prepared from 3-bromobenzoyl chloride and excess chlorobenzene in the presence of aluminium chloride by the method of Smith (*J. Amer. Chem. Soc.*, 1921, *43*, 1921) (5.9 g) and ethyl acrylate (2.1 g) reacted under the conditions described in Example 17 to give ethyl 3-(4-chlorobenzoyl)cinnamate (4.1 g) m.p. 98—99.5°. The foregoing ketone (2.1 g) was treated with the phosphorane derived from triphenyl-2-pyrrolidinoethylphosphonium bromide (2.9 g) by the method of Example 14 to give a mixture of the (*E*)- and (*Z*)-forms of ethyl 3-(3-pyrrolidino-1-(4-chlorophenyl)prop-1-enyl)cinnamate (1.6 g).

The ester mixture was hydrolysed using aqueous ethanolic sodium hydroxide solution as described in Example 14. The resulting mixture of carboxylic acids (1.5 g) was separated on a column of silica in a mixture of chloroform and methanol (1:1) to give (*E*)-3-((*Z*)-3-pyrrolidino-1-(4-chlorophenyl)prop-1-enyl)-cinnamic acid m.p. 178—181°C, and (*E*)-3-((*E*)-3-pyrrolidino-1-(4-chlorophenyl)prop-1-enyl)cinnamic acid, m.p. 193—195°C.

Example 20

3-(3-Dimethylamino)-1-(4-tolyl)prop-1-enyl)cinnamic acid

By use of the methods described in Example 17, 3-bromo-4'-methyl benzophenone (Ipatieff and Friedman, *J. Amer. Chem. Soc.*, 1939, *61*, 684) was converted into ethyl 3-(4-toluoyl)cinnamate, m.p. 86—87°, and thence into a mixture of the (*E*)- and (*Z*)-isomers of ethyl 3-(3-dimethylamino-1-(4-tolyl)prop-1-enyl)cinnamate. Hydrolysis of this ester mixture and separation of the mixture of carboxylic acids by crystallisation yielded (*E*)-3-((*E*)-3-dimethylamino-1-(4-tolyl)prop-1-enyl)cinnamic acid, m.p. 200—205°C, and (*E*)-3-((*Z*)-3-dimethylamino-1-(4-tolyl)prop-1-enyl)cinnamic acid, m.p. 200—205°C.

Example 21

3-(3-(3-Pyrrolidino-1-(4-tolyl)prop-1-enyl)phenyl)propionic acid

Ethyl 3-(4-toluoyl)cinnamate (Example 20) (3.0 g), in solution in ethyl acetate (90 ml) was shaken with hydrogen in the presence of Raney nickel catalyst until slightly more than 1 molar equivalent of hydrogen had been absorbed. After removal of the catalyst by filtration, the residue was dissolved in dichloromethane (200 ml), barrium manganate (14 g) was added and the mixture was stirred at 50° for 2 hours. The

filtered solution was evaporated to leave pure ethyl 3-(3-(4-toluoyl)phenyl)propionate as a yellow oil. (A portion hydrolysed with dilute aqueous alcohol sodium hydroxide gave the corresponding carboxylic acid, m.p. 137—138.5°).

By the methods described in Example 17, the foregoing keto-ester was converted, by way of the mixture of isomers of ethyl 3-(3-(3-pyrrolidino-1-(4-tolyl)-prop-1-enyl)phenyl)propionate, into (*E*)-3-(3-(3-pyrrolidino-1-(4-tolyl)prop-1-enyl)phenyl)propionic acid, m.p. 138—140°C, and (*Z*)-3-(3-(3-pyrrolidino-1-(4-tolyl)prop-1-enyl)phenyl)propionic acid, which was not isolated in a pure form.

Example 22

(E)-3-(6-(3-Pyrrolidino-1-(4-tolyl)prop-1E-enyl)-2-pyridyl)acrylic acid

A solution of vinyl magnesium bromide was prepared under nitrogen from vinyl bromide (5.9 g) and magnesium turnings (1.5 g) in tetrahydrofuran (50 ml). To this ice-cooled solution was added with stirring anhydrous zinc chloride (3.74 g) and the mixture stirred under $N_2$ for 2 hours at or below room, temperature. The resultant solution was decanted from the bulk of the insoluble inorganics and gradually added to an ice-cooled and stirred suspension of the keto-ester (7.38 g) in THF (50 ml). The mixture was stirred at room temperature for 44 hours by which time the reaction was almost complete. With cooling 2N aqueous HCl (50 ml) was gradually added and the mixture then poured into water (450 ml) and the product extracted with ether (600 ml) and the extract washed with water (3 × 250 ml) and brine (100 ml) and dried. Filtration and removal of the solvent *in vacuo* gave ethyl-(*E*)-3-(6-[1-(4-tolyl)-1-hydroxy-prop-2-enyl]-2-pyridyl)acrylate as a pale brown oil (8.7 g) which contained some residual THF.

To a stirred solution of the above carbinol (8.0 g) in triethylamine (30 ml) was added 4-*N,N*-dimethylaminopyridine (0.80 g) and then acetic anhydride (8 ml) and the mixture stirred at room temperature overnight. A further aliquot of acetic anhydride (4 ml) was added and the mixture stirred overnight again after which time reaction was essentially complete. With ice-cooling ethanol (30 ml) was added and the mixture stirred at room temepratture for 30 min. After concentration *in vacuo* the residue was dissolved in ether (250 ml) and the solution washed with water (50 ml), saturated sodium bicarbonate solution (2 × 50 ml) water (50 ml) and brine (40 ml) and dried. Filtration and removal of the solvent *in vacuo* gave a dark red gum (8.4 g). This crude product was then purified by dry-column chromatography using silica (250 g) and eluting with hexane/ether (3:2). Combination of the appropriate fractions and concentration *in vacuo* gave a product which when triturated with hexane and cooled gave ethyl-(*E*)-3-6-(1-(4-tolyl)-1-acetoxy-prop-2-enyl)-2-pyridyl acrylate as a pale-cream solid (2.86 g, 31%) m.pt 99°C. A sample was crystallised from hexane to give white crystals m.pt. 100°C.

The above acetate (1.825 g) was dissolved in acetonitrile (15 ml) and tetrakis triphenylphosphine palladium (O) (60 mg) added followed by triphenylphosphine (25 mg) and then pyrrolidine (0.50 ml). The mixture was then heated at 60—65°C under nitrogen with stirring for 2 hours. After cooling the mixture was poured into water (100 ml) and acidified by the addition of 2*N* aqueous hydrochloric acid (20 ml) and the solution extracted with ether (50 ml). The aqueous layer was then neutralised by the addition of a slight excess of aqueous ammonia and the product extracted with ether (100 ml) and the extract washed with water (50 ml) and brine (25 ml) and dried. Filtration and concentration *in vacuo* gave a pale-brown viscous gum (1.85 g 98%) which was a mixture of steroisomers (*E,E:EZ*=58:42) of ethyl-3-(6-(3-pyrrolidino-1-(4-tolyl)prop-1-enyl)-2-pyridyl)acrylate.

This product was mixed with 95% w/w sulphuric acid (5 ml) and heated at 130—135°C with stirring for 1 hour. The resultant solution was cooled and poured into ethanol (50 ml) and the solution heated at reflux for 1 hour. The volume was then reduced to about one half by concentration *in vacuo* and then water (100 ml) was added and the solution neutralised by the addition of a slight excess of aqueous ammonia. The product was extracted with ether (100 ml) and the extract washed with water (50 ml) and dried. Filtration and concentration *in vacuo* gave a red gum (1.17 g, 63%) which was predominantly the *E,E*-isomer ethyl-(*E*)-3-(6-(3-pyrrolidino-1-(4-tolyl)prop-1*E*-enyl)-2-acrylate.

The above *E,E*-ester (0.75 g) was hydrolysed in the usual manner by dissolving in ethanol (15 ml) and adding 1N aqueous sodium hydroxide (3 ml) and removing the alcohol by rotary evaporation *in vacuo*. To the cooled residue was added 1N aqueous sulphuric acid (3 ml) and the mixture concentrated to dryness *in vacuo*. The dry residue was extracted with hot isopropanol (3 × 4 ml) and the total extract cooled in the refigerator to give a white solid (112 mg) which was crystallised from isopropanol (6 ml) to give crystals of the title compound (42 mg).

Example 23

Isomerisation of ethyl (E)-(6-[3-pyrrolidino-1-(4-tolyl)prop-1-Z-enyl]-2-pyridyl)acrylate

The above ester (13.9 g) was mixed with 90% w/w sulphuric acid (28 ml) and the mixture heated with stirring at 130°C for 3 hours. After cooling the mixture was gradually poured into ethanol (300 ml) with cooling. The solution was then heated at reflux for 1 hour and concentrated to approx. one third of its volume *in vacuo* and then poured onto excess crushed ice. Aqueous ammonia was then added to liberate the free base which was extracted with ether (500 ml) and the extract washed with water (2 × 250 ml), brine (100 ml) and dried. Filtration and concentration *in vacuo* gave a red solid product (11.9 g, 85%) consisting of a mixture with acetonitrile (50 ml) and cooling in the refigerator gave an almost white solid which was filtered off and rinsed with a little cold acetonitrile. This material (7.5 g) was substantially pure *E,E*-isomer.

Example 24

(E)-3-(6-(3-pyrrolidino-1-(4-tolyl)prop-1E-enyl)-2-pyridyl)acrylic acid

To a solution of 2,6-dibromopyridine (52.14 g) in toluene cooled to −50°C was added with stirring under nitrogen n-butyl lithium (130 ml of 1.70M solution in hexane), maintaining the temperature at −50°C during the addition. After stirring at −50°C for a period of 2 hours a solution of 3-pyrrolidino-1-(4-tolyl)-propan-1-one (prepared from 50.72 g of the corresponding hydrochloride and azeotroped dry) in toluene (150 ml) was added at −50°C. The reaction mixture was stirred at −50° for 1.5 hours before being allowed to warm to −30°C and kept there for 1.5 hours. Hydrochloric acid (2*N*, 300 ml) was then added followed by water (800 ml). The separated aqueous layer was washed with ether and basified with 2*N* sodium hydroxide solution. The solid was filtered off and dried and crystallised twice from SVM to give 1-(4-tolyl)-1-(2-(6-bromo)-pyridyl)-3-pyrrolidino-1-propanol as white crystals (43.0 g) m.pt. 124°.

The above carbinol (43 g), ethyl acrylate (12.61 g), palladium acetate (0.518 g), triphenylphosphine (1.55 g) and *N*-ethylmorpholine (140 ml) were mixed and heated with stirring at 145°C for 5 hours. After cooling the reaction mixture was poured into water (800 ml) and the product extracted with petroleum spirit (60—80°C). The extracts were washed with water, dried and concentrated *in vacuo* to give ethyl (*E*)-3-(6-(1-(4-tolyl)-1-hydroxy-3-pyrrolidino-propyl)-2-pyridyl)acrylate as a red gum (41.6 g).

The above carbinol-ester (25.39 g) was mixed with 90% w/w sulphuric acid (50 ml) and the mixture heated with stirring at 135°C for 3 hours. The cooled mixture was poured into ethanol (760 ml) and the solution heated at reflux for 1.5 hours. The solution was concentrated to approx. one third of its volume *in vacuo* and then poured into ice (1 litre). With cooling the solution was neutralised by the addition of a slight excess of aqueous ammonia and the product extracted with ether. The extracts were washed with water, dried and concentration *in vacuo* to give a red solid (21.1 g) which consisted of a mixture of stereoisomers (*E*,*E*: *E*,*Z*=80.20) of ethyl (*E*-3-(6-(3-pyrrolidino-1-(4-tolyl)prop-1*E*,*Z*-enyl)-2-pyridyl)acrylate.

The above ester mixture (15 g) was dissolved in ethanol (250 ml) and 1*N* aqueous sodium hydroxide (60 ml) added. The alcohol was then removed by rotary evaporation *in vacuo*. The residue was neutralised by the addition of 1*N* aqueous sulphuric acid (60 ml) and the mixture taken to dryness by concentration *in vacuo*.

The residue was extracted with hot isopropanol (3 × 75 ml) and the combined extracts refrigerated. The resultant crystalline solid was filtered off and dried to give the title compound (4.57 g). A second crop (0.625 g) was obtained by concentration of the mother liquor. The combined crops were crystallised from isopropanol.

Example 25

6-(6-(3-Pyrrolidino-1-(4-tolyl)prop-1Z-enyl)-2-pyridyl)hex-5E-enoic acid

4-Carboxybutyltriphenylphosphonium bromide (12.8 g) was added under nitrogen to a stirred solution of dimsyl sodium prepared from dimethyl sulphoxide (30 ml) and sodium hydride (2.8 g of 50% oil suspension). After five minutes a solution of 2-(6-formyl-2-pyridyl)-2-(4-tolyl)-1,3-dioxolan (9.9 g) in tetrahydrofuran (20 ml) was added and the mixture was stirred at 45° for 2.5 hours. The cooled mixture was diluted with water (100 ml), washed with ether, acidified with hydrochloric acid (ice) and extracted with chloroform. The extracts were thoroughly washed with water, dried and evaporated to dryness. The residual oil (20 g) was heated in ethanol (100 ml) containing hydrochloric acid (50 ml, 2M) for 40 minutes. The crude acid recovered by evaporation was esterified with methanol/sulphuric acid and the ester was purified by distillation, b.p. 200—210°/0.2 mm. Treatment of this ester with Wittig reagent by the method of Example 1 produced a mixture of acids from which the title compound was isolated by crystallisation from ethyl acetate as off-white prisms, m.p. 118—121°.

Example 26

Antihistamine Activity

A. *In vitro* antihistamine activity: The longitudinal muscle was isolated from the intact ileum of guinea-pigs (Hartley, male 250—400 g) and placed in an organ bath under 300 mg tension. After one hour of equilibration, cumulative concentration-response curves (Van Rossum, J. M., *Arch. Int. Pharmacodyn. Ther. 143,* 299—330, 1963) to histamine were obtained. Following washing, the tissues were incubated for one hour with the test compound and then a second histamine concentration-response curve was run. Shifts to the right of the agonist concentration-response curve produced by the antagonists were used to construct Schild plots (Arunlakshana, O. and Schild, H. O., *Br. J. Pharmacol: 14,* 48—58, 1959). Regression of Log (dr-1) on Log [B], where dr is an equiactive response in the presence and absence of antagonist and [B] is the molar concentration of antagonist, allowed an estimate of $pA_2$, i.e. the negative log of the concentration of antagonist which shifts the control histamine concentration response curve 2× to the right.

Table I. Results of *in vitro* Antihistamine Assays

| | $R_1COQ$ | X | $NR_2R_3$ | $R_4$ | $pA_2$ |
|---|---|---|---|---|---|
| compound A | (E)-2-CH=CHCO$_2$H | N | | $CH_3$ | 8.6 |
| compound B | -2-CH$_2$CH$_2$CO$_2$H | N | " | $CH_3$ | 9.2 |
| | (E)-2-CH=CH.CO$_2$H | N | " | Cl | 9.0 |
| | (E)-2-CH=CHCO$_2$Et | N | " | $CH_3$ | 7.7 |
| | (E)-2-CH=CHCONH$_2$ | N | " | $CH_3$ | 8.49 |
| | (E)-2-CH=CHCO$_2$H | N | " | $OCH_3$ | 8.94 |
| | (E)-2-CH=CHCO$_2$H | N | " | $CF_3$ | 10.4 |
| | (E)-2-CH=CHCONCH$_2$CO$_2$H | N | " | $CH_3$ | 6.9 |
| | -2-CO$_2$H | CH | " | $CH_3$ | 8.1 |
| compound C | (E)-2-CH=CHCO$_2$H | N | | $CH_3$ | 8.2 |
| | -2-CO$_2$H | | | $CH_3$ | 8.9 |
| compound D | (E)-2-CH=CHCO$_2$H | CH | " | $CH_3$ | 8.8 |

B. *In vivo* Antihistaminic Activity: Guinea pigs (Hartley, male, 300—350 g) were fasted for 20 hours and then dosed p.o. or i.p. with the test compound. One hour after dosing, on an individual basis, the guinea pigs were placed in a clear plastic chamber which was saturated and continually gassed with 0.25% histamine from an aerosol nebulizer. The guinea pigs were monitored for signs of histamine anaphylaxis (e.g. cough, sneeze, strong abdominal movements, cyanoses or loss of righting). Under the test conditions, control animals collapsed on average within 33 seconds. $ED_{50}$'s for protection against histamine were calculated by probit analysis. In this test the $ED_{50}$ indicates that at that particular dose 50% of the animals were completely protected against histamine challenge at the time of testing (1 hour post-dosing). Complete protection was defined as no histamine symptoms for six minutes in the aerosol chamber (approximately 10× the collapse time of the control animals.

TABLE I

Results of Antihistamine Assays

| Compound | $ED_{50}$ (mg/kg, p.o.) |
|---|---|
| Triprolidine | 5.77 |
| A | 0.44 |
| B | 0.17 |
| C | 1.7 |
| D | 0.64 |

In addition to these results, it was found that Compound A could provide very long durations of antihistaminic activity (e.g. 11 mg/kg p.o. is the $ED_{50}$ for 24 hours protection).

Example 27

Formulations

(A)—Injection

| Ingredient | Amount per ampoule |
| --- | --- |
| Compound of formula (I) | 1.0 mg |
| Water for Injections, q.s. | 1.0 mL |

The active compound was finely ground and intimately mixed with the powdered excipients lactose, corn starch, potato starch and magnesium stearate. The formulation was then compressed to afford a tablet weighing 126 mg.

(E)—Capsule

| Ingredient | Amount per Capsule |
| --- | --- |
| Compound of Formula (I) | 1.0 mg |
| Lactose | 440.0 mg |
| Magnesium Stearate | 5.0 |

The finely ground active compound was mixed with the powdered excipients lactose, corn starch and stearic acid and packed into gelatin capsules.

(F)—Tablet

| Ingredient | Amount per Tablet |
| --- | --- |
| Compound of Formula (I) | 1.0 mg |
| Pseudoephedrine HCl | 60.0 mg |
| Lactose | 62.5 mg |
| Potato starch | 14.0 mg |
| Magnesium Stearate | 1.0 mg |
| Gelatin | 2.8 mg |

A tablet was prepared from the above formulation by the method previously described in Example 23 (D).

(G)—Syrup

| Ingredient | | Amount per 5 mL |
|---|---|---|
| Compound of Formula (I) | | 1.0 mg |
| Pseudoephedrine HCl | | 30.0 mg |
| Codeine Phosphate | | 10.0 mg |
| Guaifenesin | | 100 mg |
| Methylparaben | | 0.5 mg |
| Sodium benzoate | | 0.5 mg |
| Flavor | | q.s. |
| Color | | q.s. |
| Glycerol | | 500 mg |
| Sucrose | | 2000 mg |
| Purified Water | q.s. to | 5.0 mL |

The finely ground active compound was dissolved in the water for Injections. The solution was filtered and sterilized by autoclaving.

(B)—Suppository

| Ingredient | Amount per suppository |
|---|---|
| Compound of Formula (I) | 1.0 mg |
| Cocoa Butter, or Wecobee® Base q.s. | 2.0 g |

Wecobee is a trademark and is a hydrogenated fatty carboxylic acid.

The finely ground active compound was mixed with the melted suppository base (either Cocoa Butter or Wecobee® base), poured into molds and allowed to cool to afford the desired suppositories.

(C)—Syrup

| Ingredient | | Amount Per 5 mL |
|---|---|---|
| Compound of Formula (I) | | 1.0 mg |
| Ethanol | | 0.3 mg |
| Sucrose | | 2.0 mg |
| Methylparaben | | 0.5 mg |
| Sodium Benzoate | | 0.5 mg |
| Cherry Flavour | | q.s. |
| Coloring | | q.s. |
| Water | q.s. to | 5.0 mL |

Ethanol, sucrose, sodium benzoate, methylparaben, and flavouring were combined in 70% of the total batch quantity of water. Coloring and the active compound were dissolved in the remaining water, then the two solutions were mixed and clarified by filtration.

(D)—Tablet

| Ingredient | Amount per Tablet |
|---|---|
| Compound of Formula (I) | 1.0 mg |
| Lactose | 110.0 mg |
| Corn Starch, Pregelatinized | 2.5 mg |
| Potato Starch | 12.0 mg |
| Magnesium stearate | 0.5 mg |

A syrup containing other active ingredients in addition to a compound of formula (I) was prepared from the above ingredients by an analogous method to that described for Example 23 (C) above.

(H)—Nasal Spray

| Ingredient | | Amount per 100.0 mL |
|---|---|---|
| Compound of Formula (I) | | 1 g |
| Sodium Chloride | | 0.8 g |
| Preservative | | 0.5 g |
| Purified Water | q.s. | 100.0 mL |

The preservative was dissolved in warm purified water and after cooling to 25°—30°C the sodium chloride and the compound of formula (I) were added. The pH was then adjusted to 5.5—6.5 and purified water was added to bring the final volume to 100.0 mL.

(I)—Ophthalmic Solution

| Ingredient | | Amount per 100.0 mL |
|---|---|---|
| Compound of Formula (I) | | 0.1 g |
| Sodium Chloride | | 0.8 g |
| Preservative | | 0.5 g |
| Water for Injection | q.s. | 100.0 mL |

This formulation was prepared in a similar way to the nasal spray.

(J)—Topical Cream

| Ingredient | Amount per 100.0 mL |
|---|---|
| Compound of Formula (I) | 0.1 g |
| Emulsifying Wax, N.F. | 15.0 g |
| Mineral Oil | 5.0 g |

Ethyl (*E*)-3-(6-(3-acetoxy-1-(4-tolyl)prop-1*E*-enyl)-2-pyridyl)acrylate

A mixture of ethyl-(*E*)-3-(6-(1-acetoxy-1-(4-tolyl)prop-2-enyl)-2-pyridyl)acrylate (1.0 g) and bis(benzonitrile) palladium (II) chloride (50 mg) dissolved in acetonitrile (25 ml) was heated at reflux under nitrogen for 24 hours. Concentration *in vacuo* gave a dark red oil consisting of a mixture of the stereoisomeric acetates. Dry column chromatography of this mixture on silica (50 g) eluting with hexane/ether (1:1) and collecting fractions gave a partial separation of the isomers. From one fraction pale yellow needles of the *E,E*-isomer of the title compound (0.16 g) separated m.pt. 96—97°. A further quantity (0.14 g)

was obtained from other fractions by concentration and trituation with hexane. Both samples were identified by t.l.c., i.r. and n.m.r. as the *E,E*-isomer of the title compound.

Ethyl (*E*)-3-(6-(3-pyrrolidine-1-(4-tolyl)prop-1*E*-enyl)-2-pyridyl)acrylate

A mixture of the above *E,E*-acetate (182 mg), tetrakistriphenyl phosphine palladium (O) (6 mg), triphenylphosphine (2.5 mg) and pyrrolidine (0.05 ml) dissolved in acetonitrile (2.5 ml) was heated at 75° under nitrogen for 6 hours. After cooling the mixture was poured into water (25 ml) and acidified by the addition of 2N aqueous hydrochloric acid (5 ml). The insoluble material consisting of unreacted acetate was extracted with ether (15 ml) — this extract after drying and concentration *in vacuo* gave 100 mg of recovered acetate. The aqueous layer was then neutralised by the addition of aqueous ammonia and then extracted with ether (20 ml) and the extract washed with water (10 ml) and brine (5 ml) and dried. Concentration *in vacuo* gave the *E,E*-isomer of the title compound as a slowly crystalising oil (50 mg) which after trituration with pet. spirit (40/60°) gave a white solid (26 mg). The latter was identified by t.l.c., i.r. and n.m.r. as the *E,E*-isomer of the title compound.

**Claims for the Contracting States: BE CH DE FR GB IT LI LU NL SE**

1. A compound of the formula (I):

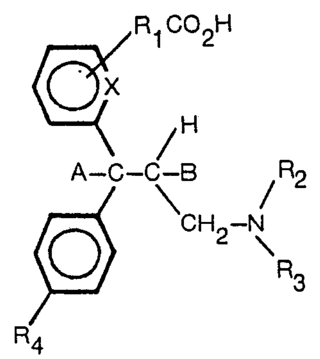

(I)

or a salt, ester or amide thereof; wherein

$R_1$ is a $C_{1-7}$ bivalent aliphatic hydrocarbon group or a single bond;

$R_2$ and $R_3$ are the same or different and are each hydrogen, $C_{1-4}$ alkyl or taken together with the nitrogen comprise a nitrogen-containing heterocyclic ring having four to six ring members;

$R_4$ is hydrogen, halogen, hydroxy, cyano, $C_{1-4}$ acyloxy, $C_{1-4}$ alkoxy or $C_{1-4}$ alkyl optionally substituted by one to three halogen atoms;

X is —N= or —CH=; and

A and B each represent hydrogen atoms or —CA—CB represents —C=C—.

2. A compound according to claim 1 of the formula (II)

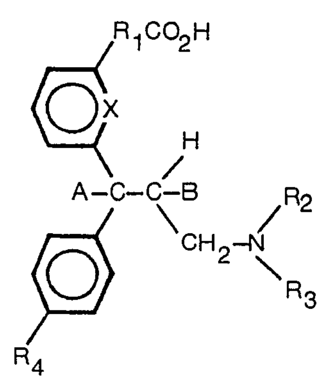

(II)

or a salt, ester of amide thereof, wherein $R_1$ to $R_4$, X, A and B are as defined in relation to claim 1.

3. A compound according to either claim 1 or claim 2 wherein $R_1$ is a group $(CH_1)_n$ where n is an integer 0 to 7, or a group $(CH_2)_aCH{=}CH(CH_2)_b$ wherein a and b are independently 0 to 5 and the sum of a and b does not exceed 5.

4. A compound according to any of claims 1 to 3 of the formula (IIIa) or (IIIb)

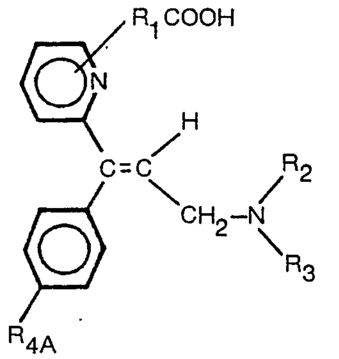

(IIIa)

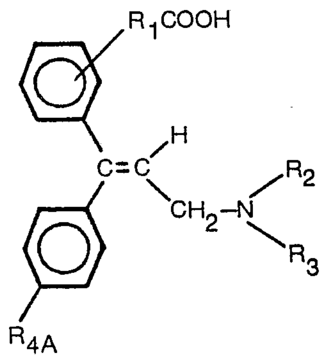

(IIIb)

or a salt, ester or amide thereof, wherein $R_1$ is $(CH_2)_n$, where n is an integer 1 to 7, or $(CH_2)_aCH:CH—(CH_2)_b$, where a and b are independently 0 to 5 and the sum of a and b does not exceed 5; $R_2$ and $R_3$ are the same or different and are hydrogen, lower alkyl (1 to 4 carbons) or taken together with the nitrogen comprise a nitrogen containing heterocyclic ring (of four to six ring members) and $R_{4A}$ is hydrogen, halogen, lower alkyl (1 to 4 carbons) or lower alkoxy (1 to 4 carbons).

5. A compound according to any of claims 1 to 4 wherein $R^2$ and $R^3$ taken together with the nitrogen form a pyrrolidino, piperidino or morpholino group.

6. A compound according to any of claims 1 to 5, wherein $R_1$ is $(CH_2)_2$ or CH=CH, $NR_2R_3$ is a pyrrolidino group or a dimethylamino group and $R_4$ is methyl, trifluoromethyl, methoxy, bromo or chloro.

7. A compound according to any one of claims 1 to 6 of the formula (IV):

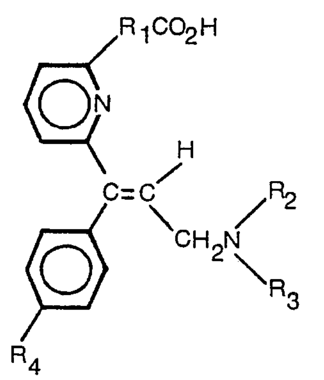

(IV)

or a salt, ester or amide thereof; wherein $R_1$ to $R_4$ are as defined in claim 1.

8. A compound according to claim 7 wherein $R_1$ is a single bond, CH=CH or $CH_2CH_2$; $NR_2R_3$ is pyrrolidino and $R_4$ is methyl or trifluoromethyl.

9. A compound according to any one of claims 1 to 8 wherein CA—CB represents a double bond and the group $CH_2NR_2R_3$ is *trans* to the X-containing ring.

10. A compound according to any one of claims 1 to 6 of the formula (V):

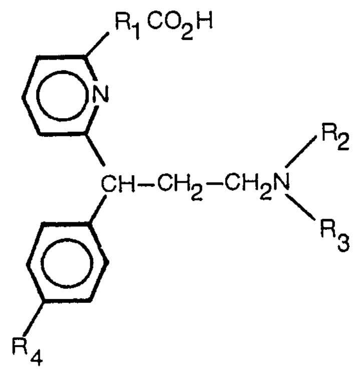

(V)

or a salt, ester or amide thereof; wherein $R_1$ to $R_4$ are as defined in claim 1.

11. A compound according to claim 10 wherein $R_1$ is a single bond, CH=CH or $CH_2CH_2$, $NR_2R_3$ is dimethylamino and $R_4$ is chlorine or bromine.

12. A compound according to claim 1 selected from:
(*E*)-3-(6-(3-pyrrolidino-1-(4-tolyl)prop-1*E*-enyl)-2-pyridyl)acrylic acid
3-(6-(3-pyrrolidino-1-(4-tolyl)prop-1*E*-enyl)-2-pyridyl)propionic acid
(*E*)-3-(6-(3-dimethylamino-1-(4-tolyl)prop-1*E*-enyl)-2-pyridyl)acrylic acid
(*E*)-3-(6-(3-pyrrolidino-1-(4-trifluoromethylphenyl)prop-1*E*-enyl)-2-pyridyl)acrylic acid
(*E*)-3-(6-(3-pyrrolidino-1-(4-methoxyphenyl)prop-1*E*-enyl)-2-pyridyl)acrylic acid
(*E*)-3-(6-(1-phenyl-3-pyrrolidinoprop-1-*E*-enyl)-2-pyridyl)acrylic acid
(*E*)-3-(6-(1-(4-chlorophenyl)-3-pyrrolidinoprop-1*E*-enyl)-2-pyridyl)acrylic acid
6-(3-pyrrolidino-1-(4-tolyl)prop-1*E*-enyl)-pyridine-2-carboxylic acid
(*E*)-3-(3-pyrrolidino-1-(4-tolyl)prop-1-enyl)benzoic acid
(*E*)-3-(3-pyrrolidino-1-(4-tolyl)prop-1-enyl)cinnamic acid
(*E*)-3-((*E*)-3-pyrrolidino-1-(4-methoxyphenyl)prop-1-enyl))cinnamic acid
(*E*)-3-((*E*)-3-dimethylamino-1-(4-tolyl)prop-1-enyl))cinnamic acid
(*E*)-3-(3-(3-pyrrolidino-1-(4-tolyl)prop-1-enyl)phenyl)propionic acid
or a salt, ester or amide thereof.

13. A method for the preparation of a compound of the formula (I), as defined in relation to claim 1 which comprises:

a) The reaction of a compound of the formula (VI)

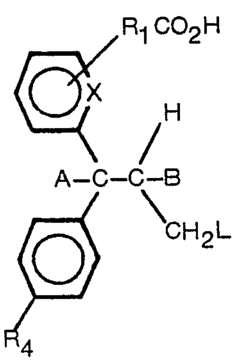

(VI)

or an ester thereof with an amine $HNR_2R_3$ wherein X, A, B and $R_1$ to $R_4$ are as defined in claim 1 and L is a leaving group.

b) When it is required to prepare a compound of the formula (I) wherein $R^1$ is $(CH_2)_0$ and A and B are hydrogen, the reaction of a compound of the formula (VII)

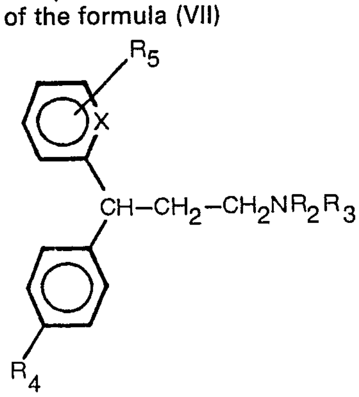

(VII)

wherein X, $R_2$, $R_3$, $R_4$ are as defined in claim 1 and $R_5$ is a halogen atom, with a $C_{1-6}$ alkyl lithium compound followed by treatment with carbon dioxide;

c) When it is required to prepare a compound of the formula (I) wherein $R^1$ is $(CH_2)_a CH{=}CH(CH_2)_b$ and a is 0, the reaction of a compound of the formula (VIII):

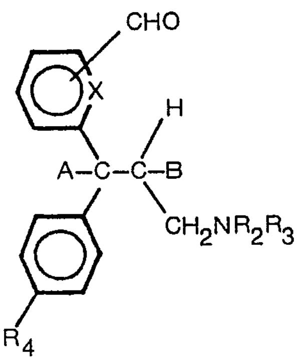

(VIII)

wherein X, A, B, $R_2$, $R_3$ and $R_4$ are as defined in claim 1 with a Wittig reagent suitable for attaching the side chain $CH{=}CH(CH_2)_bCOR_6$, wherein $COR_6$ is an acid, ester of amide group, followed by deprotection of the carboxy group if desired;

d) When it is required to prepare a compound of the formula (I) wherein CA—CB represents a double bond:

i) The reaction of an ester, amide or carboxylic acid salt of a compound of the formula (IX):

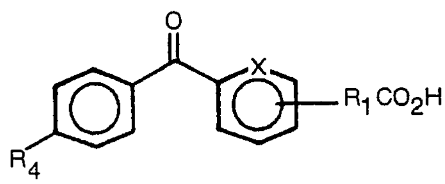

(IX)

with a Wittig reagent suitable for attaching the side chain $=CHCH_2NR_2R_3$, wherein X and $R_1$ to $R_4$ are as defined in claim 1 followed by deprotection of the carboxy group if desired;

ii) The elimination of $R^7OH$ from a compound of the formula (X):

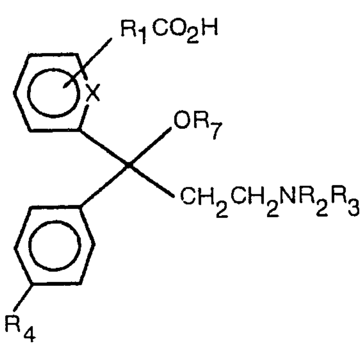

(X)

or an ester or amide thereof, wherein X, $R_1$ to $R_4$ are as defined in claim 1 and $R_7$ is hydrogen or $C_{1-4}$ acyl;

iii) The reaction of a compound of the formula (XI):

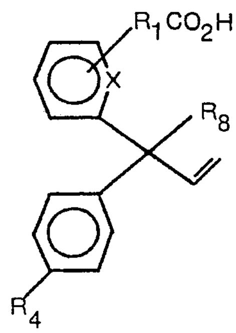

(XI)

with an amine $HNR_2R_3$, wherein X, $R_1$ to $R_4$ are as defined in claim 1 and $R_8$ is a $C_{1-4}$ acyloxy group.

e) and thereafter, optionally converting one compound of the formula (I) to another compound of the formula (I) by isomerisation of a double bond, the reduction of one or more double bonds or de-esterification of the ester group.

14. A pharmaceutical formulation which comprises a compound of the formula (I) together with one or more pharmaceutically acceptable carriers therefor and, optionally, any other therapeutic ingredients.

15. A compound of the formula (I), as defined according to any one of claims 1 to 12, for use in medicine.

16. Chemical intermediates of the formula (X) to (XII) of which formula (XII) is as follows:

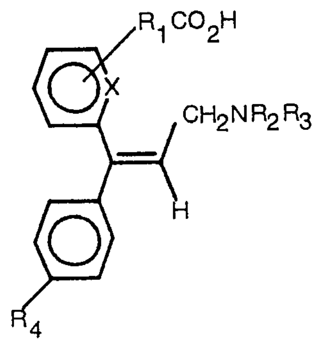

(XII)

wherein X, $R_1$ to $R_4$ are as defined in claim 1.

**Claims for the Contracting State: AT**

1. A method for the preparation of a compound of the formula (I):

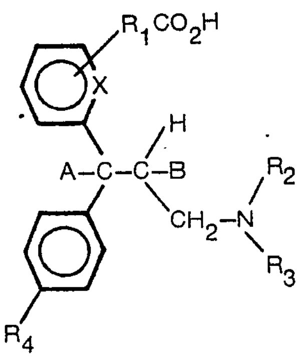

(I)

or a salt, ester or amide thereof; wherein

$R_1$ is a $C_{1-7}$ bivalent aliphatic hydrocarbon group or a single bond;

$R_2$ and $R_3$ are the same or different and are each hydrogen, $C_{1-4}$ alkyl or taken with the nitrogen comprise a nitrogen-containing heterocyclic ring having four to six ring members;

$R_4$ is hydrogen, halogen, hydroxy, cyano, $C_{1-4}$ alcyloxy, $C_{1-4}$ alkoxy or $C_{1-4}$ alkyl optionally substituted by one to three halogen atoms;

X is N= or —CH=; and

A and B each represent hydrogen atoms or —CA—CB— represents —C=C—; which comprises

a) The reaction of a compound of the formula (VI)

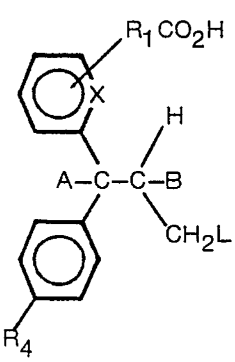

(VI)

or an ester thereof with an amine $HNR_2R_3$ wherein X, A, B and $R_1$ to $R_4$ are as defined in claim 1; and L is a leaving group.

b) When it is required to prepare a compound of the formula (I) wherein $R_1$ is $(CH_2)_0$ and A and B are hydrogen, the reaction of a compound of the formula (VII)

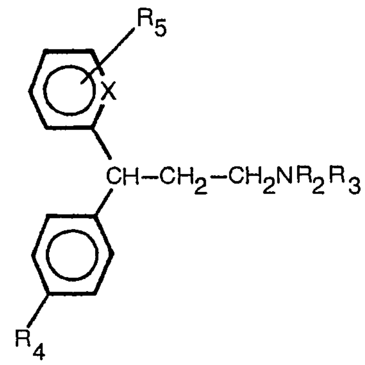

(VII)

wherein X, $R_2$, $R_3$, $R_4$ are as in claim 1 and $R_5$ is a halogen atom, with a $C_{1-6}$ alkyl lithium compound followed by treatment with carbon dioxide;

c) When it is required to prepare a compound of the formula (I) wherein $R_1$ is $(CH_2)_a$ $CH=CH(CH_2)_b$ and a is 0, the reaction of a compound of the formula (VIII):

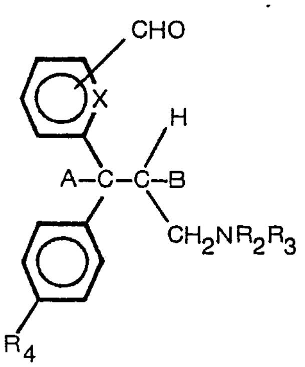

(VIII)

wherein X, A, B, $R_2$, $R_3$ and $R_4$ are as defined in claim 1 with a Wittig reagent suitable for attaching the side chain $CH=CH(CH_2)_bCOR_6$, wherein $COR_6$ is an acid, ester or amide group as hereinbefore defined, followed by deprotection of the carboxy group if desired;

d) When it is required to prepare a compound of the formula (I) wherein CA—CB represents a double bond:

i) The reaction of an ester, amide or carboxylic acid salt of a compound of the formula (IX):

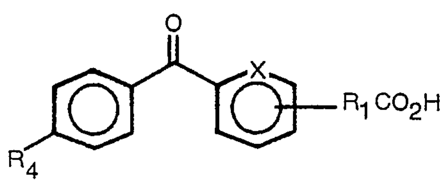

(IX)

with a Wittig reagent suitable for attaching the side chain $=CHCH_2NR_2R_3$, wherein X and $R_1$ to $R_4$ are as defined in claim 1 followed by deprotection of the carboxy group if desired;

ii) The elimination of $R_7OH$ from a compound of the formula (X):

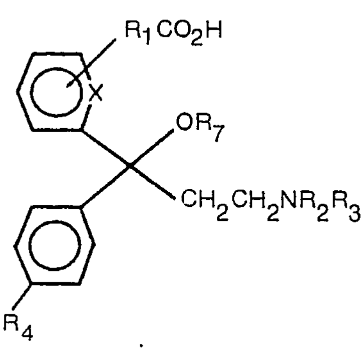

(X)

or an ester or amide thereof, wherein X, $R_1$ to $R_4$ are as defined in claim 1, $R_7$ is hydrogen or $C_{1-4}$ acyl;

iii) The reaction of a compound of the formula (XI):

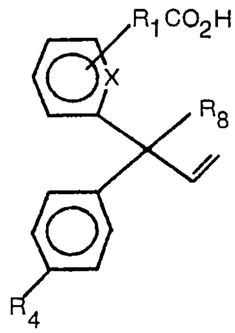

(XI)

with an amine $HNR_2R_3$, wherein X, $R_1$ to $R_4$ are as defined in claim 1 and $R_8$ is a $C_{1-4}$ acyloxy group.

e) and thereafter, optionally converting one compound of the formula (I) to another compound of the formula (I) by isomerisation of a double bond, the reduction of one or more double bonds or de-esterification of the ester group.

2. A method according to claim 1 for preparing a compound of the formula (II)

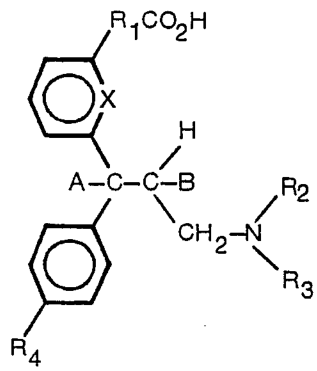

(II)

or a salt, ester or amide thereof, wherein $R_1$ to $R_4$, X, A and B are as defined in relation to claim 1.

3. A method according to either claim 1 or 2 for preparing a compound wherein $R_1$ is a group $(CH_2)_n$ where n is an integer 0 to 7, or a group $(CH_2)_a$ CH=CH $(CH_2)_b$ wherein a and b are independently 0 to 5 and the sum of a and b does not exceed 5.

4. A method according to any one of claims 1 to 3 for preparing a compound of the formula (IIIa) or (IIIb)

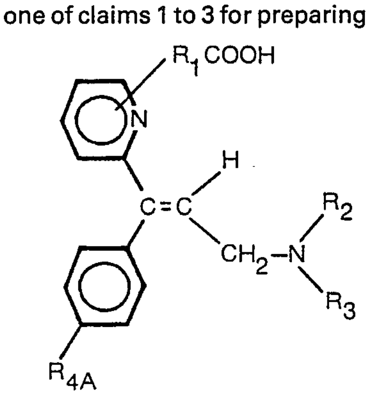

(IIIa)

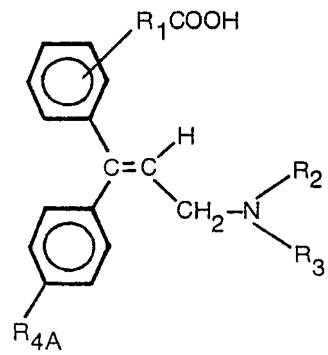

(IIIb)

or a salt, ester or amide thereof, wherein $R_1$ is $(CH_2)_n$, where n is an integer 1 to 7, or $(CH_2)_aCH{:}CH—(CH_2)_b$, where a and b are independently 0 to 5 and the sum of a and b does not exceed 5; $R_2$ and $R_3$ are the same or different and are hydrogen, lower alkyl (1 to 4 carbons) or taken together with the nitrogen comprise a nitrogen containing heterocyclic ring (of four to six ring members) and $R_{4A}$ is hydrogen, halogen, lower alkyl (1 to 4 carbons) or lower alkoxy (1 to 4 carbons).

5. A method according to any one of claims 1 to 4 for preparing a compound wherein $R^2$ and $R^3$ taken together with the nitrogen form a a pyrrolidino, piperidino or morpholino group.

6. A process according to any of claims 1 to 5, for preparing a compound wherein $R_1$ is $(CH_2)_2$ or CH=CH, $NR_2R_3$ is a pyrrolidino group or a dimethylamino group and $R_4$ is methyl, trifluoromethyl, methoxy, bromo or chloro.

7. A process according to any one of claims 1 to 6 for preparing a compound of the formula (IV):

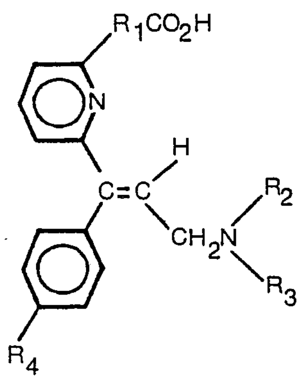

(IV)

or a salt, ester or amide thereof; wherein $R_1$ to $R_4$ are as defined in claim 1.

8. A process according to claim 7 wherein $R_1$ in the formula (IV) is a single bond, CH=CH or $CH_2CH_2$; $NR_2R_3$ is pyrrolidino and $R_4$ is methyl or trifluoromethyl.

9. A process according to any one of claims 1 to 8 wherein CA—CB represents a double bond and the group $CH_2NR_2R_3$ is *trans* to the X-containing ring.

10. A process according to any one of claims 1 to 6 for the preparation of a compound of the formula (V):

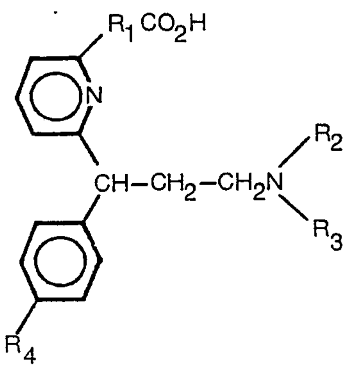

(V)

or a salt, ester or amide thereof; wherein $R_1$ to $R_4$ are as defined in claim 1.

11. A process according to claim 10 wherein $R_1$ in the formula (V) is a single bond, CH=CH or $CH_2CH_2$, $NR_2R_3$ is dimethylamino and $R_4$ is chlorine or bromine.

12. A process according to claim 1 for preparing a compound selected from:

(*E*)-3-(6-(3-pyrrolidino-1-(4-tolyl)prop-1*E*-enyl)-2-pyridyl)acrylic acid
3-(6-(3-pyrrolidino-1-(4-tolyl)prop-1*E*-enyl)-2-pyridyl)propionic acid
(*E*)-3-(6-(3-dimethylamino-1-(4-tolyl)prop-1*E*-enyl)-2-pyridyl)acrylic acid
(*E*)-3-(6-(3-pyrrolidino-1-(4-trifluoromethylphenyl)prop-1*E*-enyl)-2-pyridyl)acrylic acid
(*E*)-3-(6-(3-pyrrolidino-1-(4-methoxyphenyl)prop-1*E*-enyl)-2-pyridyl)acrylic acid
(*E*)-3-(6-(1-phenyl-3-pyrrolidinoprop-1-*E*-enyl)-2-pyridyl)acrylic acid
(*E*)-3-(6-(1-(4-chlorophenyl)-3-pyrrolidinoprop-1*E*-enyl)-2-pyridyl)acrylic acid
6-(3-pyrrolidino-1-(4-tolyl)prop-1*E*-enyl)-pyridine-2-carboxylic acid
(*E*)-3-(3-pyrrolidino-1-(4-tolyl)prop-1-enyl)benzoic acid
(*E*)-3-(3-pyrrolidino-1-(4-tolyl)prop-1-enyl)cinnamic acid
(*E*)-3-((*E*)-3-pyrrolidino-1-(4-methoxyphenyl)prop-1-enyl))cinnamic acid
(*E*)-3-((*E*)-3-dimethylamino-1-(4-tolyl)prop-1-enyl))cinnamic acid
(*E*)-3-(3-(3-pyrrolidino-1-(4-tolyl)prop-1-enyl)phenyl)propionic acid

or a salt, ester or amide thereof.

13. A pharmaceutical formulation which comprises a compound of the formula (I) together with one or more pharmaceutically acceptable carriers therefor and, optionally, any other therapeutic ingredients.

14. A process for preparing a pharmaceutical formulation as defined in claim 13 which comprises the step of bringing the active compound into association with a carrier which constitutes one or more accessory ingredients.

15. A process for the preparation of a compound of formula X,

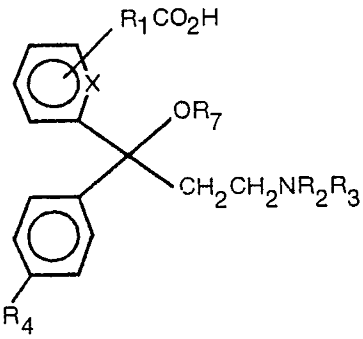

(X)

wherein $R_1$, $R_2$, $R_3$, $R_4$ and X are as defined in claim 1 and $R_7$ is hydrogen or $C_{1-4}$ acyl the process comprising either

(i) in the case where $R_7$ is hydrogen, reacting a compound of formula XX

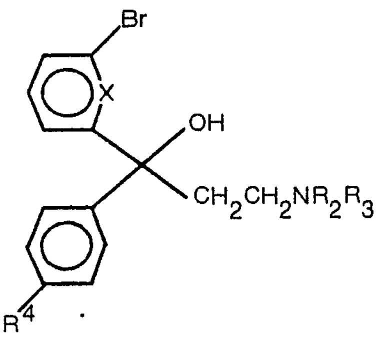

(XX)

wherein $R_2$, $R_3$, $R_4$ and X are as defined for formula X, with a compound $CH{=}CHCOR_{13}$, wherein $COR_{13}$ is an ester or amide group; or

(ii) by reacting a compound for formula XXII

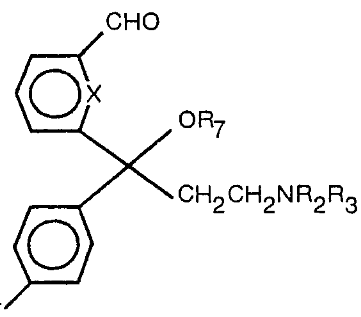

(XXII)

wherein $R_2$, $R_3$, $R_4$, $R_7$ and X are as defined for formula X, with malonic acid.

16. A process for the preparation of a compound of formula XI

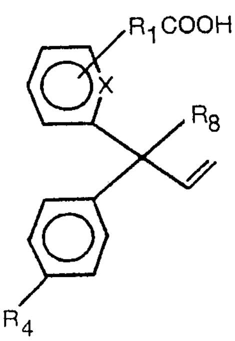

(XI)

wherein

$R_1$ is a $C_{1-7}$ bivalent aliphatic hydrocarbon group or a single bond;

$R_4$ is hydrogen, halogen, hydroxy, cyano, $C_{1-4}$ acyloxy, $C_{1-4}$ alkoxy or $C_{1-4}$ alkyl optionally substituted by one to three halogen atoms;

X is —N= or —CH=; and

$R_8$ is a $C_{1-4}$ acyloxy group.

which process comprises acylating with a $C_{1-4}$ acylating agent the corresponding compound wherein $R_8$ is a hydroxy group.

17. A process as claimed in claim 1 process step (e), wherein the compound of Formula I is also a compound of Formula XII

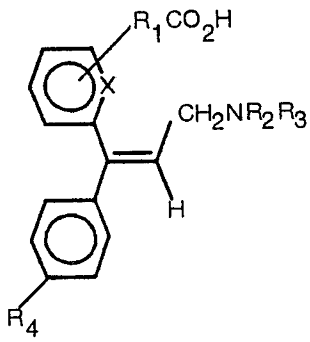

(XII)

wherein $R_1$, $R_2$, $R_3$, $R_4$ and X are as defined for Formula I.

**Patentansprüche für die Vertragsstaaten: BE CH DE FR GB IT LI LU NL SE**

1. Verbindung der Formel (I)

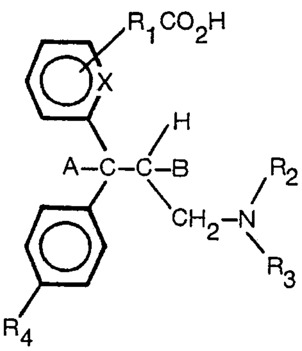

(I)

oder deren Salz, Ester oder Amid, worin

$R_1$ eine $C_{1-7}$-bivalente aliphatische Kohlenwasserstoffgruppe oder eine Einfachbindung bedeutet;

$R_2$ und $R_3$ gleich oder verschieden sind und jedes Wasserstoff, und $C_{1-4}$-Alkyl repräsentiert oder zusammen mit dem Stickstoff einen 4- bis 6-gliedrigen, Stickstoff enthaltenden. heterocyclischen Ring bedeuten;

$R_4$ Wasserstoff, Halogen, Hydroxy, Cyano, $C_{1-4}$-Acyloxy, $C_{1-4}$-Alkoxy oder $C_{1-4}$-Alkyl bedeutet, gegebenenfalls substituiert durch 1 bis 3 Halogenatome;

X —N= oder —CH= bedeutet; und

A und B jedes Wasserstoffatome darstellt oder —CA—CB —C=C— darstellt.

2. Verbindung nach Anspruch 1 mit der allgemeinen Formel (II)

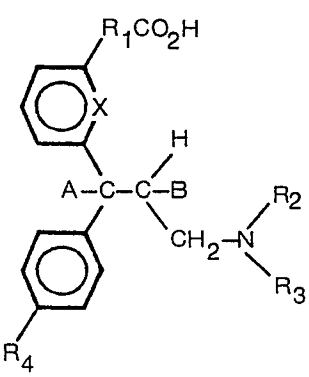

(II)

oder deren Salz, Ester oder Amid, worin $R_1$ bis $R_4$, X, A und B die in Anspruch 1 genannte Bedeutung haben.

3. Verbindung nach Anspruch 1 oder 2, wobei $R_1$ eine Gruppe $(CH_2)_n$ ist, wobei n eine ganze Zahl zwischen 0 bis 7 ist, oder eine Gruppe $(CH_2)_a$ $CH=CH(CH_2)_b$, worin a und b unabhängig voneinander 0 bis 5 sind und die Summe von a und b 5 nicht übersteigt.

4. Verbindung nach einem der Ansprüche 1 bis 3 gemäß den Formeln (IIIa) oder (IIIb),

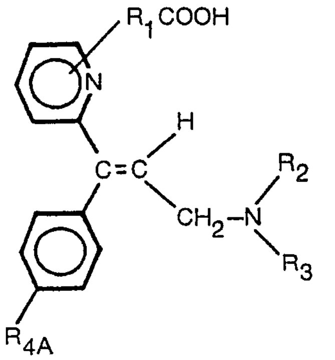

(IIIa)

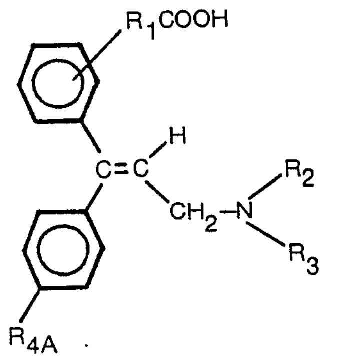

(IIIb)

oder deren Salz, Ester oder Amid, worin $R_1$ $(CH_2)_n$ ist, wobei n eine ganze Zahl zwischen 1 bis 7 ist, oder $(CH_2)_aCH=CH—(CH_2)_b$, wobei a und b unabhängig voneinander 0 bis 5 sind und die Summe von a und b 5 nicht übersteigt; $R_2$ und $R_3$ sind gleich oder verschieden und sind Wasserstoff, Niedrigalkyl (1 bis 4 Kohlenstoffatome) oder bilden zusammen mit dem Stickstoffatom einen Stickstoff enthaltenden heterocyclischen Ring (4- bis 6-gliedrig) und $R_{4A}$ bedeutet Wasserstoff, Halogen, Niederalkyl (1 bis 4 Kohlenstoffatome) oder Niederalkoxy (1 bis 4 Kohlenstoffatome).

5. Verbindung nach einem der Ansprüche 1 bis 4, wobei $R^2$ und $R^3$ zusammen mit dem Stickstoff eine Pyrrolidino-, Piperidino- oder Morpholinogruppe bilden.

6. Verbindung nach einem der Ansprüche 1 bis 5, wobei $R_1$ $(CH_2)_2$ oder CH=CH bedeutet, $NR_2R_3$ eine Pyrrolidinogruppe oder eine Dimethylaminogruppe bedeutet und $R_4$ Methyl, Trifluormethyl, Methoxy, Brom oder Chlor bedeutet.

7. Verbindung der allgemeinen Formel (IV) nach einem der Ansprüche 1 bis 6

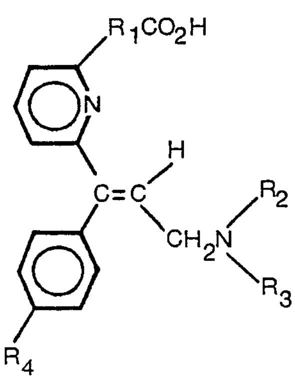

(IV)

oder deren Salz, Ester oder Amid, worin $R_1$ bis $R_4$ die in Anspruch 1 genannte Bedeutung haben.

8. Verbindung nach Anspruch 7, worin $R_1$ eine Einfachbindung, CH=CH oder $CH_2CH_2$ bedeutet; $NR_2R_3$ bedeutet Pyrrolidino und $R_4$ bedeutet Methyl oder Trifluormethyl.

9. Verbindung nach einem der Ansprüche 1 bis 8, wobei CA—CB eine Doppelbindung darstellt und die Gruppe $CH_2NR_2R_3$ in trans-Stellung zum X enthaltenden Ring steht.

10. Verbindung der Formel (V) nach einem der Ansprüche 1 bis 6

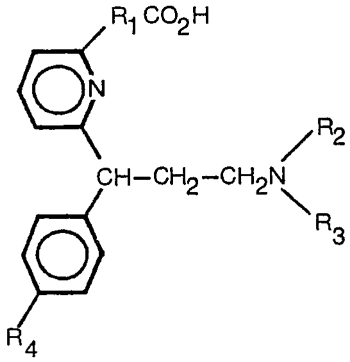

(V)

oder deren Salz, Ester oder Amid, worin $R_1$ bis $R_4$ die in Anspruch 1 genannte Bedeutung haben.

11. Verbindung nach Anspruch 10, worin $R_1$ eine Einfachbindung, CH=CH oder $CH_2CH_2$ bedeutet, $NR_2R_3$ bedeutet Dimethylamino und $R_4$ bedeutet Chlor oder Brom.

12. Verbindung nach Anspruch 1, ausgewählt aus:
(E)-3-(6-(3-Pyrrolidino-1-(4-tolyl)-prop-1E-enyl)-2-pyridyl)-acrylsäure,
3-(6-(3-Pyrrolidino-1-(4-tolyl)-prop-1E-enyl)-2-pyridyl)-propionsäure,
(E)-3-(6-(3-Dimethylamino-1-(4-tolyl)-prop-1E-enyl)-2-pyridyl)-acrylsäure,
(E)-3-(6-(3-Pyrrolidino-1-(4-trifluormethylphenyl)-prop-1E-enyl)-2-pyridyl)-acrylsäure,
(E)-3-(6-(3-Pyrrolidino-1-(4-methoxyphenyl)-prop-1E-enyl)-2-pyridyl)-acrylsäure,
(E)-3-(6-(1-Phenyl-3-pyrrolidinoprop-1-E-enyl)-2-pyridyl)-acrylsäure,
(E)-3-(6-(1-(4-Chlorphenyl)-3-pyrrolidinoprop-1E-enyl)-2-pyridyl)-acrylsäure
6-(3-Pyrrolidino-1-(4-tolyl)-prop-1E-enyl)-pyridin-2-carbonsäure
(E)-3-(3-Pyrrolidino-1-(4-tolyl)-prop-1-enyl)-benzoesäure,
(E)-3-(3-Pyrrolidino-1-(4-tolyl)-prop-1-enyl)-zimtsäure,
(E)-3-((E)-3-Pyrrolidino-1-(4-methoxyphenyl)-prop-1-enyl)-zimtsäure,
(E)-3-((E)-3-Dimethylamino-1-(4-tolyl)-prop-1-enyl))-zimtsäure,
(E)-3-(3-(3-Pyrrolidino-1-(4-tolyl)-prop-1-enyl)-phenyl)-propionsäure
oder deren Salz, Ester oder Amid.

13. Verfahren zur Herstellung einer Verbindung gemäß Formel (I) nach Anspruch 1, umfassend:
a) Reaktion einer Verbindung der Formel (VI)

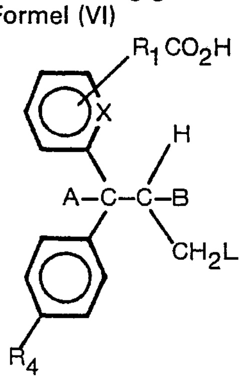

(VI)

oder deren Ester mit einem Amin $HNR_2R_3$, worin X, A, B und $R_1$ bis $R_4$ die in Anspruch 1 genannte Bedeutung haben und L eine Austrittsgruppe bedeutet;

b) falls eine Verbindung gemäß Formel (I) hergestellt werden soll, worin $R^1$ $(CH_2)_0$ und A und B Wasserstoff sind, Reaktion einer Verbindung der Formel (VII),

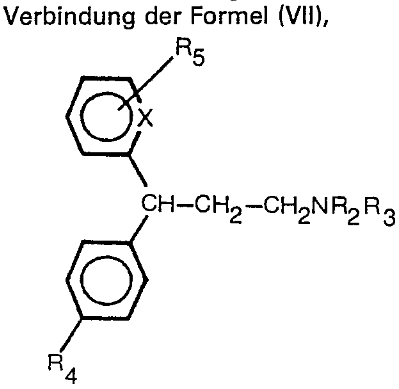

(VII)

worin X, $R_2$, $R_3$, $R_4$ die in Anspruch 1 genannte Bedeutung haben und $R_5$ ein Halogenatom bedeutet, mit einer $C_{1-6}$-Alkyllithiumverbindung und nachfolgender Behandlung mit Kohlendioxid;

c) falls eine Verbindung nach Formel (I) hergestellt werden soll, worin $R^1$ $(CH_2)_a$ $CH=CH(CH_2)_b$ und a 0 ist, Reaktion einer Verbindung der Formel (VIII)

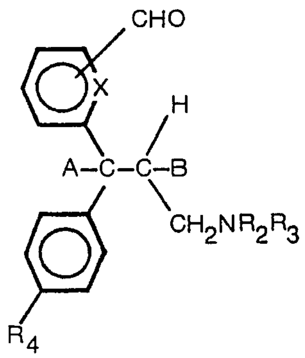

(VIII)

worin X, A, B, $R_2$, $R_3$ und $R_4$ die in Anspruch 1 genannte Bedeutung haben mit einem geeigneten Wittig-Reagens zum Verknüpfen der Seitenkette $CH=CH(CH_2)_bCOR_6$, worin $COR_6$ eine Säure, Ester- oder Amidgruppe ist, mit — falls gewünscht — nachfolgender Deprotektion der Carboxygruppe;

d) falls eine Verbindung nach Formel (I) hergestellt werden soll, worin CA—CB eine Doppelbindung bedeutet:

(i) Reaktion eines Esters, Amids oder Carbonsäuresalzes einer Verbindung nach Formel (IX):

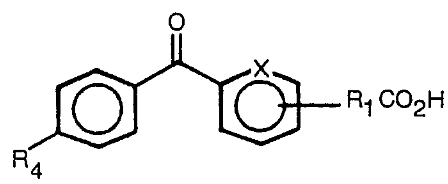

(IX)

mit einem geeigneten Wittig-Reagens für die Verknüpfung der Seitenkette $=CHCH_2NR_2R_3$, worin X und $R_1$ bis $R_4$ die in Anspruch 1 gegebene Bedeutung haben, mit — falls gewünscht — nachfolgender Deprotektion der Carboxygruppe;

(ii) Eliminierung von $R^7OH$ aus einer Verbindung nach Formel (X):

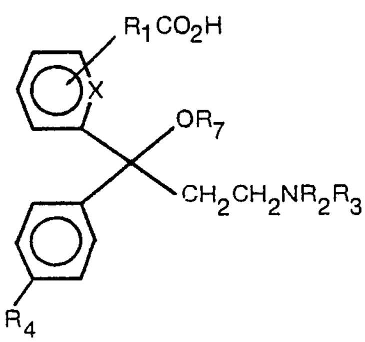

(X)

oder deren Ester oder Amid, worin X, $R_1$ bis $R_4$ die in Anspruch 1 genannte Bedeutung haben und $R_7$ Wasserstoff oder $C_{1-4}$-Acyl bedeutet;

(iii) Reaktion einer Verbindung gemäß Formel (XI):

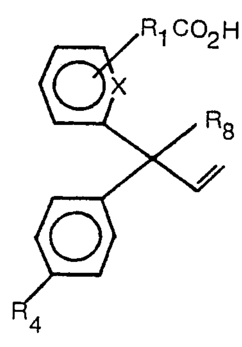

(XI)

mit einem Amin $HNR_2R_3$, worin X, $R_1$ bis $R_4$ die in Anspruch 1 genannte Bedeutung haben und $R_8$ eine $C_{1-4}$-Acyloxygruppe bedeutet.

e) gegebenenfalls anschließendes Umwandeln einer Verbindung nach Formel (I) in eine andere Verbindung der Formel (I) durch Isomerisierung einer Doppelbindung, Reduzierung einer oder mehrerer Doppelbindungen oder Entesterung der Estergruppe.

14. Pharmazeutische Zubereitung, umfassend eine Verbindung der Formel (I) zusammen mit einem oder mehreren pharmazeutisch annehmbaren Trägern und gegebenenfalls anderen therapeutischen Bestandteilen.

15. Verwendung einer Verbindung gemäß Formel (I), nach einem der Ansprüche 1 bis 12, zur medizinischen Anwendung.

16. Chemische Zwischenstufen gemäß den Formeln (X) bis (XII), wobei Formel (XII) lautet:

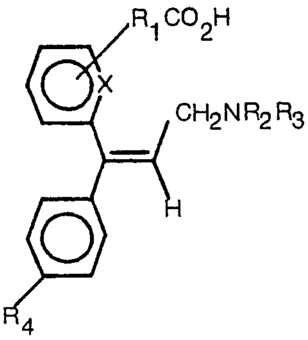

(XII)

worin X, $R_1$ bis $R_4$ die in Anspruch 1 genannte Bedeutung haben.

**Patentansprüche für den Vertragsstaat: AT**

1. Verfahren zur Herstellung einer Verbindung der Formel (I):

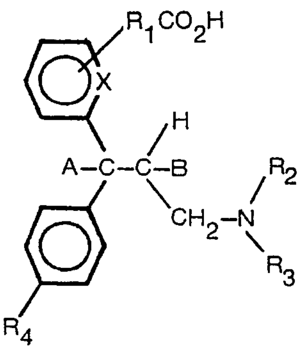

(I)

oder deren Salz, Ester oder Amid, worin

$R_1$ eine $C_{1-7}$ bivalente aliphatische Kohlenwasserstoffgruppe oder eine Einfachbindung bedeutet;

$R_2$ und $R_3$ gleich oder verschieden sind und jedes Wasserstoff, und $C_{1-4}$-Alkyl repräsentiert oder zusammen mit dem Stickstoff einen 4- bis 6-gliedrigen, Stickstoff enthaltenden heterocyclischen Ring bedeuten;

$R_4$ Wasserstoff, Halogen, Hydroxy, Cyano, $C_{1-4}$-Acyloxy, $C_{1-4}$-Alkoxy oder $C_{1-4}$-Alkyl bedeutet, gegebenenfalls substituiert durch 1 bis 3 Halogenatome;

X —N= oder —CH= bedeutet; und

A und B jedes Wasserstoffatome darstellt oder —CA—CB —C=C— darstellt;

umfassend:

a) Reaktion einer Verbindung der Formel (VI)

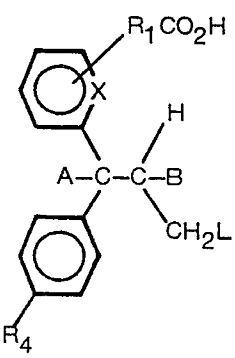

(VI)

oder deren Ester mit einem Amin $HNR_2R_3$, worin X, A, B und $R_1$ bis $R_4$ die in Anspruch 1 genannte Bedeutung haben, und L eine Austrittsgruppe bedeutet;

b) falls eine Verbindung gemäß Formel (I) hergestellt werden soll, worin $R^1$ $(CH_2)_0$ und A und B Wasserstoff sind, Reaktion einer Verbindung der Formel (VII)

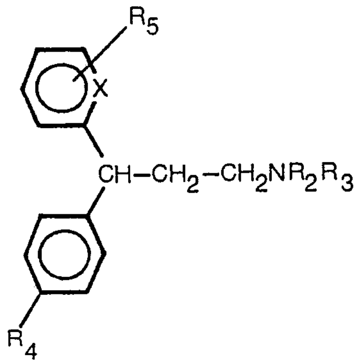

(VII)

worin X, $R_2$, $R_3$, $R_4$ die in Anspruch 1 genannte Bedeutung haben und $R_5$ ein Halogenatom bedeutet, mit einer $C_{1-16}$-Alkyllithiumverbindung und nachfolgender Behandlung mit Kohlendioxid;

c) falle eine Verbindung nach Formel (I) hergestellt werden soll, worin $R^1$ $(CH_2)_a$ $CH{=}CH(CH_2)_b$ und a 0 ist, Reaktion einer Verbindung der Formel (VIII)

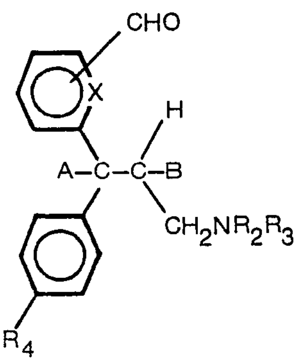

(VIII)

worin X, A, B, $R_2$, $R_3$ und $R_4$ die in Anspruch 1 genannte Bedeutung haben mit einem geeigneten Wittig-Reagens zum Verknüpfen der Seitenkette $CH{=}CH(CH_2)_bCOR_6$, worin $COR_6$ eine vorstehend definierte Säure, Ester- oder Amidgruppe ist, mit — falls gewünscht — nachfolgender Deprotektion der Carboxygruppe;

d) falls eine Verbindung nach Formel (I) hergestellt werden soll, worin CA—CB eine Doppelbindung bedeutet:

i) Reaktion eines Esters, Amids oder Carbonsäuresalzes einer Verbindung nach Formel (IX):

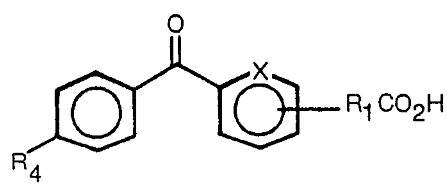

(IX)

mit einem geeigneten Wittig-Reagens für die Verknüpfung der Seitenkette $=CHCH_2NR_2R_3$, worin X und $R_1$ bis $R_4$ die in Anspruch 1 gegebene Bedeutung haben, mit — falls gewünscht — nachfolgender Deprotektion der Carboxygruppe;

ii) Eliminierung von $R^7OH$ aus einer Verbindung nach Formel (X):

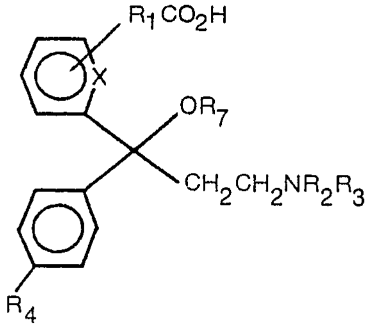

(X)

oder deren Ester oder Amid, worin X, $R_1$ bis $R_4$ die in Anspruch 1 genannte Bedeutung haben und $R_7$ Wasserstoff oder $C_{1-4}$-Acyl bedeutet;

iii) Reaktion einer Verbindung gemäß Formel (XI):

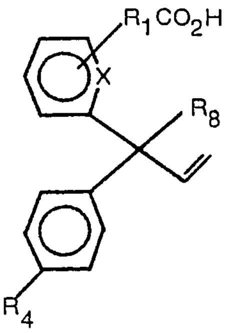

(XI)

mit einem Amin $HNR_2R_3$, worin X, $R_1$ bis $R_4$ die in Anspruch 1 genannte Bedeutung haben und $R_8$ eine $C_{1-4}$-Acyloxygruppe bedeutet;

e) gegebenenfalls anschließendes Umwandeln einer Verbindung nach Formel (I) in eine andere Verbindung der Formel (I) durch Isomerisierung einer Doppelbindung, Reduzierung einer oder mehrerer Doppelbindungen oder Entesterung der Estergruppe.

2. Verfahren nach Anspruch 1 zur Herstellung einer Verbindung nach Formel (II)

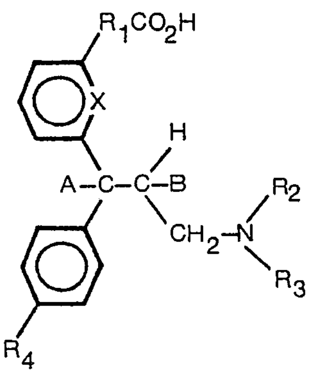

(II)

oder deren Salz, Ester oder Amid, worin $R_1$ bis $R_4$, X, A und B die in Anspruch 1 genannte Bedeutung haben.

3. Verfahren nach einem der Ansprüche 1 oder 2 zur Herstellung einer Verbindung, worin $R_1$ eine Gruppe $(CH_2)_n$ bedeutet, worin n eine ganze Zahl von 0 bis 7 ist, oder eine Gruppe $(CH_2)_a$ $CH{=}CH(CH_2)_b$ bedeutet, worin a und b unabhängig voneinander 0 bis 5 sind und die Summe von a und b 5 nicht übersteigt.

4. Verfahren nach einem der Ansprüche 1 bis 3 zur Herstellung einer Verbindung nach Formel (IIIa) oder (IIIb)

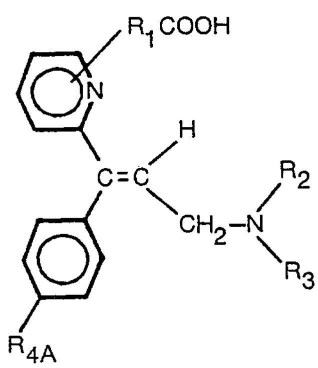

(IIIa)

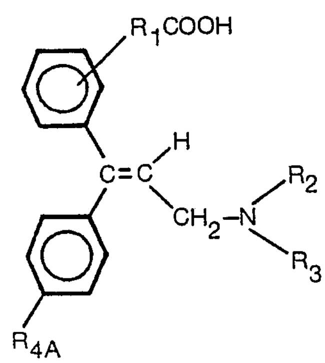

(IIIb)

oder deren Salz, Ester oder Amid, worin $R_1$ $(CH_2)_n$ ist, wobei n eine ganze Zahl zwischen 1 bis 7 ist, oder $(CH_2)_aCH{=}CH{-}(CH_2)_b$, bedeutet, wobei a und b unabhängig voneinander 0 bis 5 sind und die Summe von a und b 5 nicht übersteigt; $R_2$ und $R_3$ sind gleich oder verschieden und sind Wasserstoff, Niedrigalkyl (1 bis 4 Kohlenstoffatome) oder bilden zusammen mit dem Stickstoffatom einen Stickstoff enthaltenden heterocyclischen Ring (4- bis 6-gliedrig) und $R_{4A}$ bedeutet Wasserstoff, Halogen, Niederalkyl (1 bis 4 Kohlenstoffatome) oder Niederalkoxy (1 bis 4 Kohlenstoffatome).

5. Verfahren nach einem der Ansprüche 1 bis 4 zur Herstellung einer Verbindung, worin $R^2$ und $R^3$ zusammen mit dem Stickstoff eine Pyrrolidino-, Piperidino- oder Morpholinogruppe bilden.

6. Verfahren nach einem der Ansprüche 1 bis 5 zur Herstellung einer Verbindung, worin $R_1$ $(CH_2)_2$ oder CH=CH bedeutet, $NR_2R_3$ eine Pyrrolidinogruppe oder eine Dimethylaminogruppe bedeutet und $R_4$ Methyl, Trifluormethyl, Methoxy, Brom oder Chlor bedeutet.

7. Verfahren nach einem der Ansprüche 1 bis 6 zur Herstellung einer Verbindung nach Formel (IV):

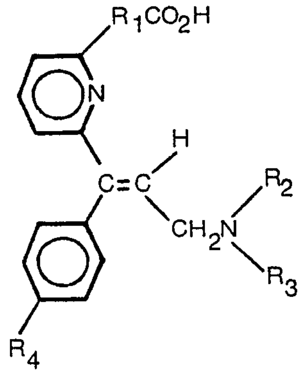

(IV)

oder deren Salz, Ester oder Amid, worin $R_1$ bis $R_4$ die in Anspruch 1 genannte Bedeutung haben.

8. Verfahren nach Anspruch 7, worin $R_1$ in Formel (IV) eine Einfachbindung, CH=CH oder $CH_2CH_2$ bedeutet; $NR_2R_3$ bedeutet Pyrrolidino und $R_4$ bedeutet Methyl oder Trifluormethyl.

9. Verfahren nach einem der Ansprüche 1 bis 8, worin CA—CB eine Doppelbindung darstellt und die Gruppe $CH_2NR_2R_3$ in trans-Stellung zum X enthaltenden Ring steht.

10. Verfahren nach einem der Ansprüche 1 bis 6 zur Herstellung einer Verbindung der Formel (V)

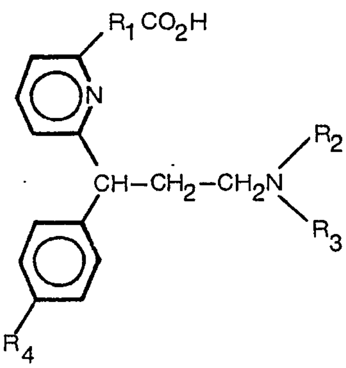

(V)

oder deren Salz, Ester oder Amid, worin $R_1$ bis $R_4$ die in Anspruch 1 gegebene Bedeutung haben.

11. Verfahren nach Anspruch 10, worin $R_1$ in Formel (V) eine Einfachbindung, CH=CH oder $CH_2CH_2$ bedeutet, $NR_2R_3$ bedeutet Dimethylamino und $R_4$ bedeutet Chlor oder Brom.

12. Verfahren nach Anspruch 1 zur Herstellung einer Verbindung, ausgewählt aus:
(E)-3-(6-(3-Pyrrolidino-1-(4-tolyl)-prop-1E-enyl)-2-pyridyl)-acrylsäure,
3-(6-(3-Pyrrolidino-1-(4-tolyl)-prop-1E-enyl)-2-pyridyl)-propionsäure,
(E)-3-(6-(3-Dimethylamino-1-(4-tolyl)-prop-1E-enyl)-2-pyridyl)-acrylsäure,
(E)-3-(6-(3-Pyrrolidino-1-(4-trifluormethylphenyl)-prop-1E-enyl)-2-pyridyl)-acrylsäure,
(E)-3-(6-(3-Pyrrolidino-1-(4-methoxyphenyl)-prop-1E-enyl)-2-pyridyl)-acrylsäure,
(E)-3-(6-(1-Phenyl-3-pyrrolidinoprop-1-E-enyl)-2-pyridyl)-acrylsäure,
(E)-3-(6-(1-(4-Chlorphenyl)-3-pyrrolidinoprop-1E-enyl)-2-pyridyl)-acrylsäure
6-(3-Pyrrolidino-1-(4-tolyl)-prop-1E-enyl)-pyridin-2-carbonsäure
(E)-3-(3-Pyrrolidino-1-(4-tolyl)-prop-1-enyl)-benzoesäure,
(E)-3-(3-Pyrrolidino-1-(4-tolyl)-prop-1-enyl)-zimtsäure,
(E)-3-((E)-3-Pyrrolidino-1-(4-methoxyphenyl)-prop-1-enyl)-zimtsäure,
(E)-3-((E)-3-Dimethylamino-1-(4-tolyl)-prop-1-enyl))-zimtsäure,
(E)-3-(3-(3-Pyrrolidino-1-(4-tolyl)-prop-1-enyl)-phenyl)-propionsäure,
oder deren Salz, Ester oder Amid.

13. Pharmazeutische Zubereitung, umfassend eine Verbindung der Formel (I) zusammen mit einem oder mehreren pharmazeutisch annehmbaren Trägern und gegebenenfalls anderen therapeutischen Bestandteilen.

14. Verfahren zur Herstellung einer pharmazeutischen Zubereitung nach Anspruch 13, bei dem die aktive Verbindung mit einem Träger zusammengebracht wird, der einen oder mehrere Nebenbestandteil(e) darstellt.

15. Verfahren zur Herstellung einer Verbindung der Formel (X):

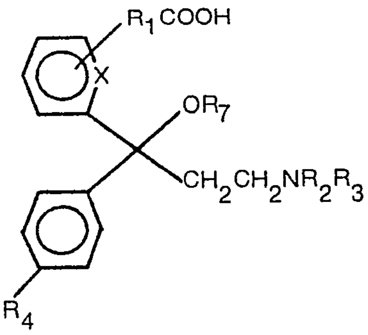

(X)

worin $R_1$, $R_2$, $R_3$, $R_4$ und X die in Anspruch 1 definierte Bedeutung haben und $R_7$ Wasserstoff oder $C_{1-4}$-Acyl bedeutet, bei dem entweder

(i) für den Fall, daß $R_7$ Wasserstoff ist, eine Verbindung der Formel XX

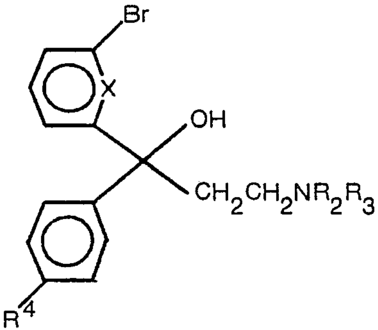

(XX)

worin $R_2$, $R_3$, $R_4$ und X die in Formel (X) gegebene Bedeutung haben, umgesetzt wird mit einer Verbindung $CH=CHCOR_{13}$, worin $COR_{13}$ eine Ester- oder Amidgruppe ist; oder

(ii) eine Verbindung der Formel XXII

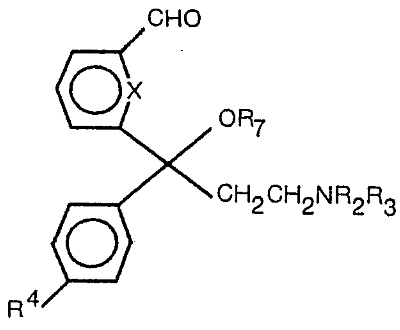

(XXII)

worin $R_2$, $R_3$, $R_4$, $R_7$ und X die in Formel (X) gegebene Bedeutung haben, umgesetzt wird mit Malonsäure.

16. Verfahren zur Herstellung einer Verbindung der Formel (XI)

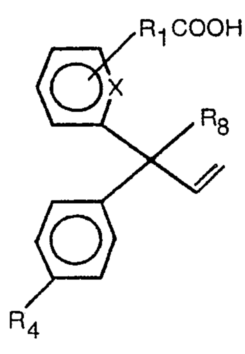

(XI)

worin

$R_1$ eine $C_{1-7}$-bivalente aliphatische Kohlenwasserstoffgruppe oder eine Einfachbindung bedeutet;

$R_4$ Wasserstoff, Halogen, Hydroxy, Cyano, $C_{1-4}$-Acyloxy, $C_{1-4}$-Alkoxy oder $C_{1-4}$-Alkyl bedeutet, gegebenenfalls substituiert durch 1 bis 3 Halogenatome;

X —N= oder —CH= bedeutet; und

$R_8$ eine $C_{1-4}$-Acyloxygruppe bedeutet;

die entsprechende Verbindung, worin $R_8$ eine Hydroxygruppe bedeutet, mit einem $C_{1-4}$-Acylierungsmittel acyliert wird.

17. Verfahren nach Anspruch 1, Verfahrensschritt (e), worin die Verbindung der Formel (I) eine Verbindung nach Formel (XII) ist,

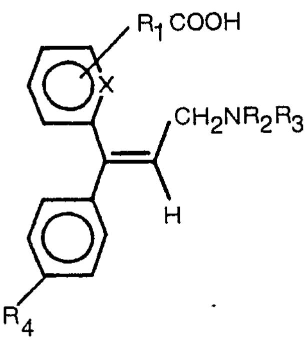

(XII)

worin $R_1$, $R_2$, $R_3$, $R_4$ und X die in Formel (I) gegebene Bedeutung haben.

**Revendications pour les Etats contractants: BE CH DE FR GB IT LI LU NL SE**

1. Composé de formule (I):

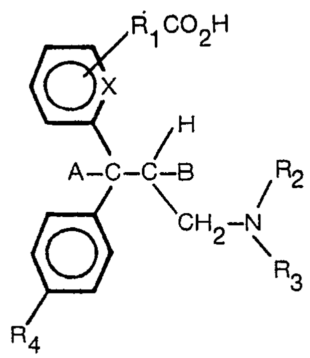

(I)

ou un sel, ester ou amide de celui-ci, où

$R_1$ est un radical hydrocarboné aliphatique bivalent en $C_{1-7}$ ou une liaison simple;

$R_2$ et $R_3$ sont identiques ou différents et sont chacun un atome d'hydrogène ou radical $C_{1-4}$alcoyle ou bien, pris ensemble avec l'atome d'azote, forment un radical hétérocyclique azoté comptant quatre à six chaînons;

$R_4$ est un atome d'hydrogène ou d'halogène ou radical hydroxyle, cyano, $C_{1-4}$acyloxy, $C_{1-4}$alcoxy ou $C_{1-4}$alcoyle éventuellement substitué par 1 à 3 atomes d'halogène;

X est —N= ou —CH=; et

A et B représentent chacun un atome d'hydrogène ou bien —CA—CB représente —C=C—.

2. Composé suivant la revendication 1 de formule (II)

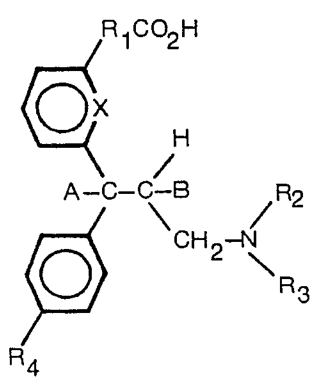

(II)

ou un sel, ester ou amide de celui-ci, où $R_1$ à $R_4$, X, A et B sont tels que définis à propos de la revendication 1.

3. Composé suivant la revendication 1 ou 2, dans lequel $R_1$ est un radical $(CH_2)_n$ où n est un entier de 0 à 7 ou un radical $(CH_2)_aCH{=}CH(CH_2)_b$ où a et b sont indépendamment 0 à 5 et la somme de a et b n'excède pas 5.

4. Composé suivant l'une quelconque des revendications 1 à 3 de formule (IIIa) ou (IIIb)

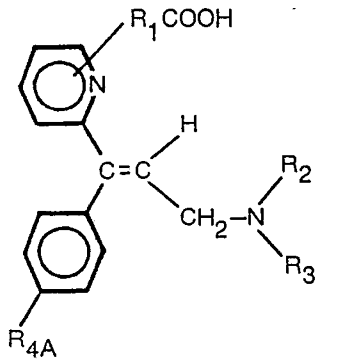

(IIIa)

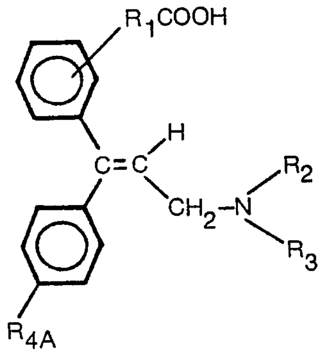

(IIIb)

ou un sel, ester ou amide de celui-ci, où $R_1$ est $(CH_2)_n$, où n est un entier de 1 à 7 ou bien $(CH_2)_aCH{:}CH(CH_2)_b$, où a et b sont indépendamment 0 à 5 et la somme de a et b n'excède pas 5; $R_2$ et $R_3$ sont identiques ou différents et sont des atomes d'hydrogène ou radicaux alcoyle inférieurs (de 1 à 4 atomes de carbone) ou bien, pris ensemble avec l'atome d'azote, forment un radical hétérocyclique azoté (de 4 à 6 chaînons); et $R_{4A}$ est un atome d'hydrogène ou d'halogène ou radical alcoyle inférieur (de 1 à 4 atomes de carbone) ou alcoxy inférieur (de 1 à 4 atomes de carbone).

5. Composé suivant l'une quelconque des revendications 1 à 4, où $R^2$ et $R^3$ pris ensemble avec l'atome d'azote forment un radical pyrrolidino, pipéridino ou morpholino.

6. Composé suivant l'une quelconque des revendications 1 à 5, dans lequel $R_1$ est $(CH_2)_2$ ou $CH{=}CH$, $NR_2R_3$ est un radical pyrrolidino ou diméthylamino et $R_4$ est un radical méthyle, trifluorométhyle, méthoxy, bromo ou chloro.

7. Composé suivant l'une quelconque des revendications 1 à 6 de formule (IV):

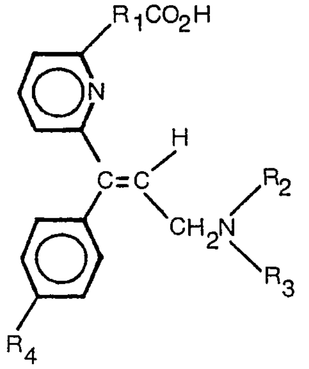

(IV)

ou un sel, ester ou amide de celui-ci; où $R_1$ à $R_4$ sont tels que définis dans la revendication 1.

8. Composé suivant la revendication 7, où $R_1$ est une liaison simple ou un radical $CH{=}CH$ ou $CH_2CH_2$; $NR_2R_3$ est un radical pyrrolidino et $R_4$ est un radical méthyle ou trifluorométhyle.

9. Composé suivant l'une quelconque des revendications 1 à 8 où CA—CB représente une double liaison, et le radical $CH_2NR_2R_3$ est en *trans* par rapport au cycle contenant X.

10. Composé suivant l'une quelconque des revendications 1 à 6 de formule (V):

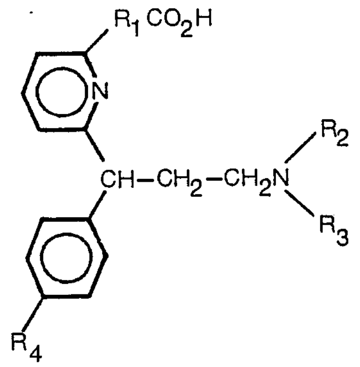

(V)

ou un sel, ester ou amide de celui-ci; où $R_1$ à $R_4$ sont tels que définis dans la revendication 1.

11. Composé suivant la revendication 10, où $R_1$ est une liaison unique ou un radical CH=CH ou $CH_2CH_2$, $NR_2R_3$ est un radical diméthylamino et $R_4$ est un radical chloro ou bromo.

12. Composé suivant la revendication 1, choisi parmi:

l'acide (*E*)-3-(6-(3-pyrrolidino-1-(4-tolyl)prop-1*E*-ényl)-2-pyridyl)acrylique
l'acide 3-(6-(3-pyrrolidino-1-(4-tolyl)prop-1*E*-ényl)-2-pyridyl)propionique
l'acide (*E*)-3-(6-(3-diméthylamino-1-(4-tolyl)prop-1*E*-ényl)-2-pyridyl)acrylique
l'acide (*E*)-3-(6-(3-pyrrolidino-1-(4-trifluorométhylphényl)prop-1*E*-ényl)-2-pyridyl)acrylique
l'acide (*E*)-3-(6-(3-pyrrolidino-1-(4-méthoxyphényl)prop-1*E*-ényl)-2-pyridyl)acrylique
l'acide (*E*)-3-(6-(1-phényl-3-pyrrolidinoprop-1-*E*-ényl)-2-pyridyl)acrylique
l'acide (*E*)-3-(6-(1-(4-chlorophényl)-3-pyrrolidinoprop-1*E*-ényl)-2-pyridyl)acrylique
l'acide 6-(3-pyrrolidino-1-(4-tolyl)prop-1*E*-ényl)pyridine-2-carboxylique
l'acide (*E*)-3-(3-pyrrolidino-1-(4-tolyl)prop-1-ényl)benzoïque
l'acide (*E*)-3-(3-pyrrolidino-1-(4-tolyl)prop-1-ényl)cinnamique
l'acide (*E*)-3-((*E*)-3-pyrrolidino-1-(4-méthoxyphényl)prop-1-ényl))cinnamique
l'acide (*E*)-3-((*E*)-3-diméthylamino-1-(4-tolyl)prop-1-ényl))cinnamique
l'acide (*E*)-3-(3-(3-pyrrolidino-1-(4-tolyl)prop-1-ényl)phényl)propionique

ou un sel, ester ou amide de celui-ci.

13. Procédé de préparation d'un composé de formule (I), tel que défini à propos de la revendication 1, qui comprend:

a) la réaction d'un composé de formule (VI)

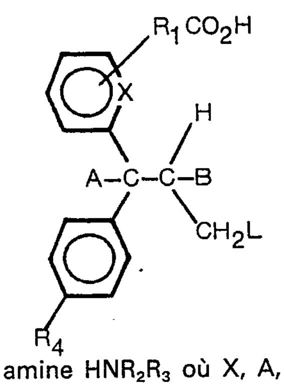

(VI)

ou d'un ester de celui-ci avec une amine $HNR_2R_3$ où X, A, B et $R_1$ à $R_4$ sont tels que définis dans la revendication 1 et L est un radical partant;

b) lorsqu'il est requis de préparer un composé de formule (I) où $R^1$ est $(CH_2)_0$ et A et B sont des atomes d'hydrogène, la réaction d'un composé de formule (VII)

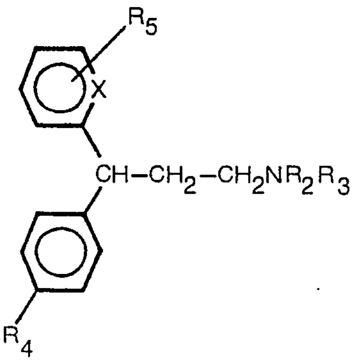

(VII)

où X, $R_2$, $R_3$ et $R_4$ sont tels que définis dans la revendication 1 et $R_5$ est un atome d'halogène, avec un $C_{1-6}$alcoyllithium, suivie d'un traitement au moyen de dioxyde de carbone;

c) lorsqu'il est requis de préparer un composé de formule (I) où $R_1$ est $(CH_2)_aCH{=}CH(CH_2)_b$ et a est 0, la réaction d'un composé de formule (VIII):

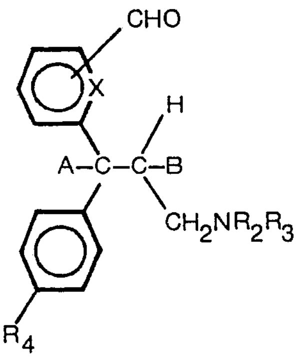

(VIII)

où X, $R_2$, $R_3$, $R_4$, A et B sont tels que définis dans la revendication 1, avec un réactif de Wittig propre à fixer la chaîne latérale $CH{=}CH(CH_2)_bCOR_6$, où $COR_6$ est un radical acide, ester ou amide tel que défini ci-dessus, suivie de la déprotection du radical carboxyle si la chose est souhaitée;

d) lorsqu'il est requis de préparer un composé de formule (I) où CA—CB représente une double liaison:

i) la réaction d'un ester, amide ou sel d'acide carboxylique d'un composé de formule (IX):

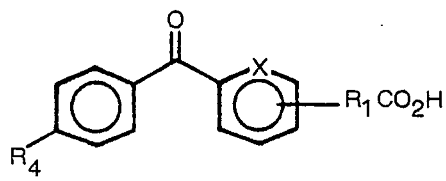

(IX)

avec un réactif de Wittig propre à fixer la chaîne latérale $=CHCH_2NR_2R_3$ où X et $R_1$ à $R_4$ sont tels que définis dans la revendication 1, suivie de la déprotection du radical carboxyle si la chose est souhaitée;

ii) l'élimination de $R_7OH$ d'un composé de formule (X):

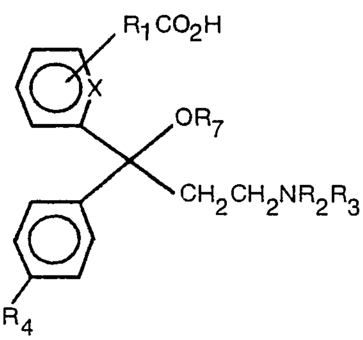

(X)

ou d'un ester ou amide de celui-ci, où X et $R_1$ à $R_4$ sont tels que définis dans la revendication 1 et $R_7$ est un atome d'hydrogène ou radical $C_{1-4}$acyle;

iii) la réaction d'un composé de formule (XI):

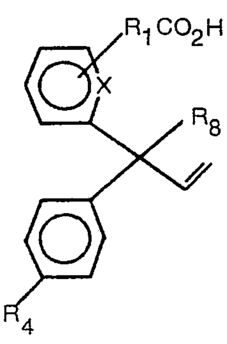

(XI)

avec une amine $HNR_2R_3$ où X et $R_1$ à $R_4$ sont tels que définis dans la revendication 1 et $R_8$ est un radical $C_{1-4}$acyloxy;

e) et ensuite, la conversion facultative d'un composé de formule (I) en un autre composé de formule (I) par isomérisation d'une double liaison, réduction d'une ou plusieurs doubles liaisons ou déestérification du radical ester.

14. Composition pharmaceutique, qui comprend un composé de formule (I) conjointement avec un ou plusieurs excipients pharmaceutiquement acceptables et, facultativement, d'autres constituants thérapeutiques quelconques.

15. Composé de formule (I) tel que défini dans l'une quelconque des revendications 1 à 12, à utiliser en médecine.

16. Intermédiaires chimiques de formules (X) à (XII), parmi lesquelles la formule (XII) est la suivante:

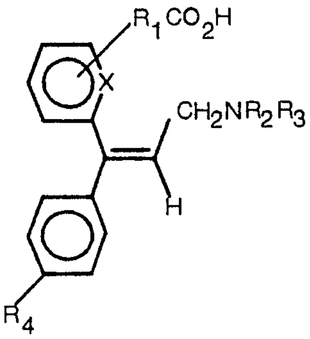

(XII)

où X, $R_1$ à $R_4$ sont tels que définis dans la revendication 1.

**Revendications pour l'Etat contractant: AT**

1. Procédé de préparation d'un composé de formule (I):

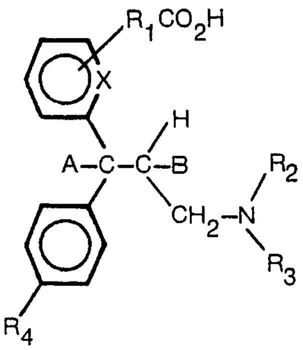

(I)

ou d'un sel, ester ou amide de celui-ci, où

$R_1$ est un radical hydrocarboné aliphatique bivalent en $C_{1-7}$ ou une liaison simple;

$R_2$ et $R_3$ sont identiques ou différents et sont chacun un atome d'hydrogène ou radical $C_{1-4}$alcoyle ou bien, pris ensemble avec l'atome d'azote, forment un radical hétérocyclique azoté comptant quatre à six chaînons;

$R_4$ est un atome d'hydrogène ou d'halogène ou radical hydroxyle, cyano, $C_{1-4}$acyloxy, $C_{1-4}$alcoxy ou $C_{1-4}$alcoyle éventuellement substitué par 1 à 3 atomes d'halogène;

X est —N= ou —CH=; et

A et B représentent chacun un atome d'hydrogène ou bien —CA—CB représente —C=C—;

qui comprend

a) la réaction d'un composé de formule (VI)

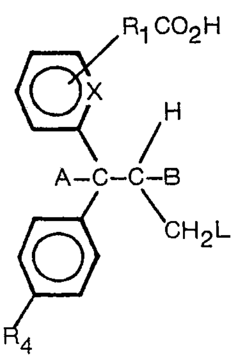

(VI)

ou d'un ester de celui-ci avec une amine $HNR_2R_3$ où X, A, B et $R_1$ à $R_4$ sont tels que définis dans la revendication 1 et L est un radical partant;

b) lorsqu'il est requis de préparer un composé de formule (I) où $R^1$ est $(CH_2)_0$ et A et B sont des atomes d'hydrogène, la réaction d'un composé de formule (VII)

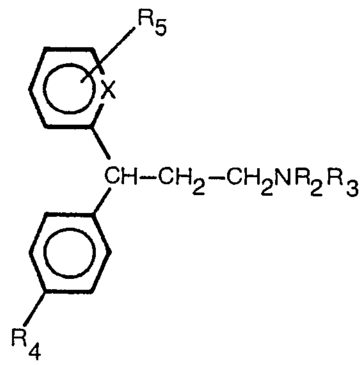

(VII)

où X, $R_2$, $R_3$ et $R_4$ sont tels que définis dans la revendication 1 et $R_5$ est un atome d'halogène, avec un $C_{1-6}$alcoyllithium, suivie d'un traitement au moyen de dioxyde de carbone;

c) lorsqu'il est requis de préparer un composé de formule (I) où $R_1$ est $(CH_2)_aCH{=}CH(CH_2)_b$ et a est 0, la réaction d'un composé de formule (VIII):

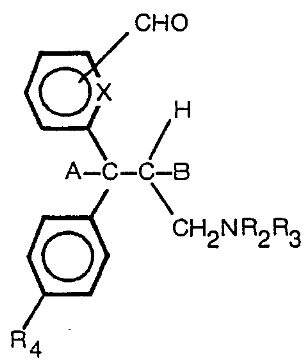

(VIII)

où X, $R_2$, $R_3$, $R_4$, A et B sont tels que définis dans la revendication 1, avec un réactif de Wittig propre à fixer la chaîne latérale $CH{=}CH(CH_2)_bCOR_6$, où $COR_6$ est un radical acide, ester ou amide tel que défini ci-dessus, suivie de la déprotection du radical carboxyle si la chose est souhaitée;

d) lorsqu'il est requis de préparer un composé de formule (I) où CA—CB représente une double liaison:

i) la réaction d'un ester, amide ou sel d'acide carboxylique d'un composé de formule (IX):

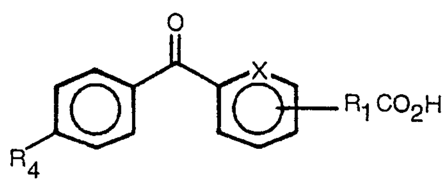

(IX)

avec un réactif de Wittig propre à fixer la chaîne latérale $=CHCH_2NR_2R_3$ où X et $R_1$ à $R_4$ sont tels que définis dans la revendication 1, suivie de la déprotection du radical carboxyle si la chose est souhaitée;

ii) l'élimination de $R_7OH$ d'un composé de formule (X):

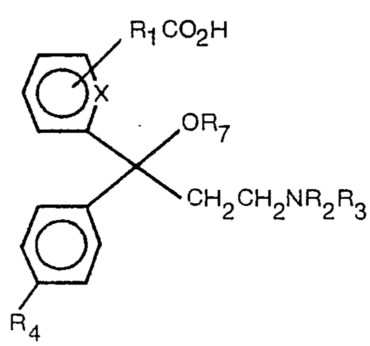

(X)

ou d'un ester ou amide de celui-ci, où X et $R_1$ à $R_4$ sont tels que définis dans la revendication 1 et $R_7$ est un atome d'hydrogène ou radical $C_{1-4}$acyle;

iii) la réaction d'un composé de formule (XI):

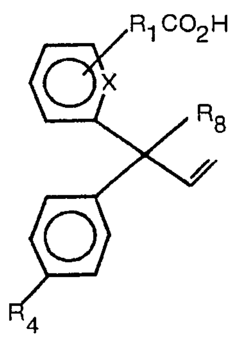

(XI)

avec une amine $HNR_2R_3$ où X et $R_1$ à $R_4$ sont tels que définis dans la revendication 1 et $R_8$ est un radical $C_{1-4}$acyloxy;

e) et ensuite, la conversion facultative d'un composé de formule (I) en un autre composé de formule (I) par isomérisation d'une double liaison, réduction d'une ou plusieurs doubles liaisons ou déestérification du radical ester.

2. Procédé suivant la revendication 1 de préparation d'un composé de formule (II)

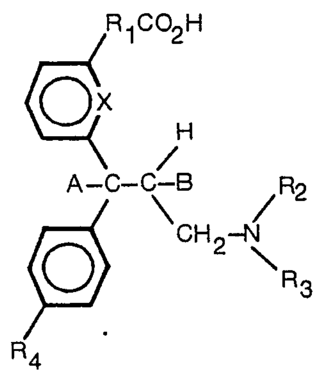

(II)

ou un sel, ester ou amide de celui-ci, où $R_1$ à $R_4$, X, A et B sont tels que définis à propos de la revendication 1.

3. Procédé suivant la revendication 1 ou 2 de préparation d'un composé où $R_1$ est un radical $(CH_2)_n$ où n est un entier de 0 à 7 ou un radical $(CH_2)_aCH{=}CH(CH_2)_b$ où a et b sont indépendamment 0 à 5 et la somme de a et b n'excède pas 5.

4. Procédé suivant l'une quelconque des revendications 1 à 3 de préparation d'un composé de formule (IIIa) ou (IIIb)

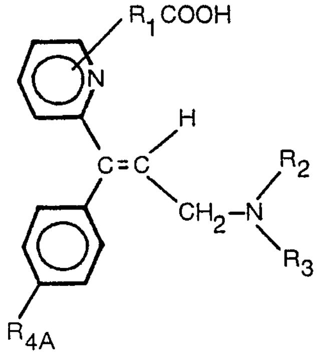

(IIIa)

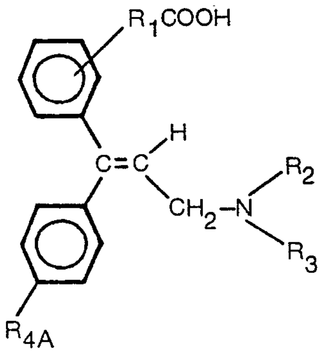

(IIIb)

ou d'un sel, ester ou amide de celui-ci, où $R_1$ est $(CH_2)_n$, où n est un entier de 1 à 7 ou bien $(CH_2)_aCH{:}CH(CH_2)_b$, où a et b sont indépendamment 0 à 5 et la somme de a et b n'excède pas 5; $R_2$ et $R_3$ sont identiques ou différents et sont des atomes d'hydrogène ou radicaux alcoyle inférieurs (de 1 à 4 atomes de carbone) ou bien, pris ensemble avec l'atome d'azote, forment un radical hétérocyclique azoté (de 4 à 6

chaînons); et $R_{4A}$ est un atome d'hydrogène ou d'halogène ou radical alcoyle inférieur (de 1 à 4 atomes de carbone) ou alcoxy inférieur (de 1 à 4 atomes de carbone).

5. Procédé suivant l'une quelconque des revendications 1 à 4 de préparation d'un composé où $R^2$ et $R^3$ pris ensemble avec l'atome d'azote forment un radical pyrrolidino, pipéridino ou morpholino.

6. Procédé suivant l'une quelconque des revendications 1 à 5 de préparation d'un composé où $R_1$ est $(CH_2)_2$ ou $CH{=}CH$, $NR_2R_3$ est un radical pyrrolidino ou diméthylamino et $R_4$ est un radical méthyle, trifluorométhyle, méthoxy, bromo ou chloro.

7. Procédé suivant l'une quelconque des revendications 1 à 6 de préparation d'un composé de formule (IV):

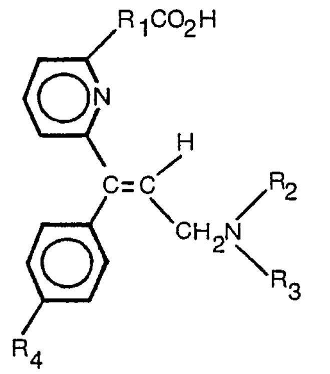

(IV)

ou d'un sel, ester ou amide de celui-ci; où $R_1$ à $R_4$ sont tels que définis dans la revendication 1.

8. Procédé suivant la revendication 7, dans lequel $R_1$ dans la formule (IV) est une liaison simple ou le radical $CH{=}CH$ ou $CH_2CH_2$; $NR_2R_3$ est un radical pyrrolidino et $R_4$ est un radical méthyle ou trifluorométhyle.

9. Procédé suivant l'une quelconque des revendications 1 à 8, dans lequel CA—CB représente une double liaison, et le radical $CH_2NR_2R_3$ est en *trans* par rapport au cycle contenant X.

10. Procédé suivant l'une quelconque des revendications 1 à 6 de préparation d'un composé de formule (V):

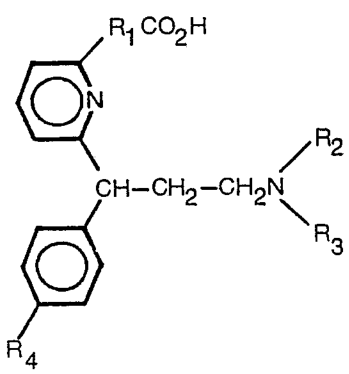

(V)

ou d'un sel, ester ou amide de celui-ci; où $R_1$ à $R_4$ sont tels que définis dans la revendication 1.

11. Procédé suivant la revendication 10, où $R_1$ dans la formule (V) est une liaison simple ou un radical $CH{=}CH$ ou $CH_2CH_2$, $NR_2R_3$ est un radical diméthylamino et $R_4$ est un radical chloro ou bromo.

12. Procédé suivant la revendication 1 de préparation d'un composé choisi parmi:
l'acide (*E*)-3-(6-(3-pyrrolidino-1-(4-tolyl)prop-1*E*-ényl)-2-pyridyl)acrylique
l'acide 3-(6-(3-pyrrolidino-1-(4-tolyl)prop-1*E*-ényl)-2-pyridyl)propionique
l'acide (*E*)-3-(6-(3-diméthylamino-1-(4-tolyl)prop-1*E*-ényl)-2-pyridyl)acrylique
l'acide (*E*)-3-(6-(3-pyrrolidino-1-(4-trifluorométhylphényl)prop-1*E*-ényl)-2-pyridyl)acrylique
l'acide (*E*)-3-(6-(3-pyrrolidino-1-(4-méthoxyphényl)prop-1*E*-ényl)-2-pyridyl)acrylique
l'acide (*E*)-3-(6-(1-phényl-3-pyrrolidinoprop-1-*E*-ényl)-2-pyridyl)acrylique
l'acide (*E*)-3-(6-(1-(4-chlorophényl)-3-pyrrolidinoprop-1*E*-ényl)-2-pyridyl)acrylique
l'acide 6-(3-pyrrolidino-1-(4-tolyl)prop-1*E*-ényl)pyridine-2-carboxylique
l'acide (*E*)-3-(3-pyrrolidino-1-(4-tolyl)prop-1-ényl)benzoïque
l'acide (*E*)-3-(3-pyrrolidino-1-(4-tolyl)prop-1-ényl)cinnamique
l'acide (*E*)-3-((*E*)-3-pyrrolidino-1-(4-méthoxyphényl)prop-1-ényl))cinnamique
l'acide (*E*)-3-((*E*)-3-diméthylamino-1-(4-tolyl)prop-1-ényl))cinnamique
l'acide (*E*)-3-(3-(3-pyrrolidino-1-(4-tolyl)prop-1-ényl)phényl)propionique
ou d'un sel, ester ou amide de celui-ci.

13. Composition pharmaceutique, qui comprend un composé de formule (I) conjointement avec un ou plusieurs excipients pharmaceutiquement acceptables et, facultativement, d'autres constituants thérapeutiques quelconques.

14. Procédé de préparation d'une composition pharmaceutique telle que définie dans la revendication 13, qui comprend le stade de mettre le composé actif en association avec un excipient qui comprend un ou plusieurs constituants accessoires.

15. Procédé de préparation d'un composé de formule X

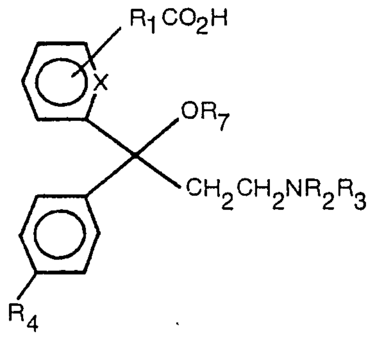

(X)

où $R_1$, $R_2$, $R_3$, $R_4$ et X sont tels que définis dans la revendication 1 et $R_7$ est un atome d'hydrogène ou radical $C_{1-4}$acyle, le procédé comprenant soit

(i) au cas où $R_7$ est un atome d'hydrogène, la réaction d'un composé de formule XX

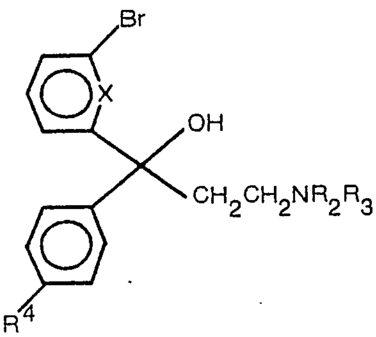

(XX)

où $R_2$, $R_3$, $R_4$ et X sont tels que définis à propos de la formule X, avec un composé CH=CHCOR$_{13}$ où COR$_{13}$ est un radical ester ou amide soit

(ii) la réaction d'un composé de formule XXII

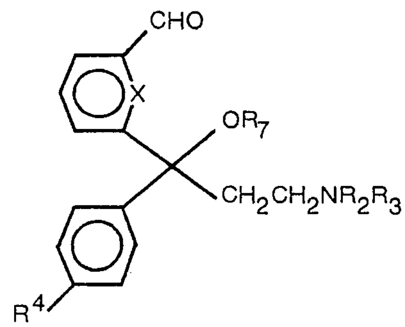

(XXII)

où $R_2$, $R_3$, $R_4$, $R_7$ et X sont tels que définis à propos de la formule X, avec l'acide malonique.

16. Procédé de préparation d'un composé de fomrule XI

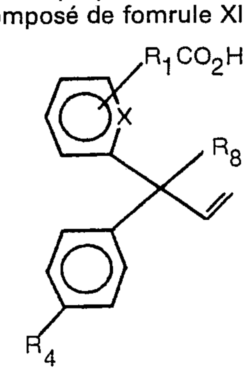

(XI)

où

$R_1$ est un radical hydrocarboné aliphatique bivalent en $C_{1-7}$ ou une liaison simple;

$R_4$ est un atome d'hydrogène ou d'halogène ou radical hydroxyle, cyano, $C_{1-4}$acyloxy, $C_{1-4}$alcoxy ou $C_{1-4}$alcoyle éventuellement substitué par 1 à 3 atomes d'halogène;

X est —N= ou —CH=; et

$R_8$ est un radical $C_{1-4}$acyloxy

lequel procédé comprend l'acylation du composé correspondant où $R_8$ est un radical hydroxyle au moyen d'un agent de $C_{1-4}$acylation.

17. Procédé suivant la revendication 1, stade opératoire (e), où le composé de formule I est un composé de formule XII

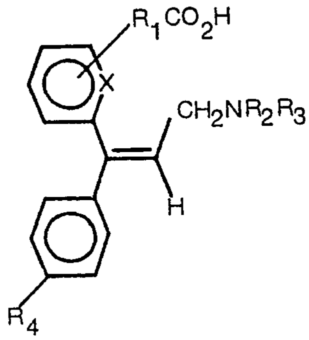

(XII)

où $R_1$, $R_2$, $R_3$, $R_4$ et X sont tels que définis pour la formule I.